# EUROPÄISCHE PATENTANMELDUNG

(11) **EP 4 491 135 A1**
(43) Veröffentlichungstag der Anmeldung: **15.01.2025**
(21) Anmeldenummer: 24187953.5
(22) Anmeldetag: 11.07.2024
(51) Int. Cl.: A61B 17/16, A61B 17/84

(54) **PRÄPARATIONSINSTRUMENT ZUM PRÄPARIEREN EINES KNOCHENS**

(30) Priorität: 12.07.2023 DE 102023118429
(71) Anmelder: Aesculap AG, 78532 Tuttlingen (DE)
(72) Erfinder: Stern, Andreas, 78087 Mönchweiler (DE); Gerlitz, Jana, 91154 Roth (DE)
(74) Vertreter: Hoeger, Stellrecht & Partner Patentanwälte mbB

(57) **Zusammenfassung**

Die Erfindung betrifft ein Präparationsinstrument zum Präparieren eines Knochens vor der Verankerung einer Knochenschraube, insbesondere einer Pedikelschraube, im Knochen, wobei das Präparationsinstrument einen eine Instrumentenlängsachse definierenden Instrumentenschaft, ein distales Ende und einen vom Instrumentenschaft separaten und vollständig trennbaren Führungsdraht, insbesondere in Form eines K-Drahts, umfasst und wobei das Präparationsinstrument ausgebildet ist zum Verankern des Führungsdrahts im Knochen, wobei der Führungsdraht mit dem Präparationsinstrument koppelbar ausgebildet ist, insbesondere zum Führen des Präparationsinstruments beim Präparieren des Knochens, und dass das Präparationsinstrument einen Führungsdrahtbewegungsmechanismus umfasst zum Bewegen des Führungsdrahts relativ zum distalen Ende in distaler und/oder proximaler Richtung.

## Beschreibung

Die vorliegende Erfindung betrifft ein Präparationsinstrument zum Präparieren eines Knochens vor der Verankerung einer Knochenschraube, insbesondere einer Pedikelschraube, im Knochen, wobei das Präparationsinstrument einen eine Instrumentenlängsachse definierenden Instrumentenschaft, ein distales Ende und einen vom Instrumentenschaft separaten und vollständig trennbaren Führungsdraht, insbesondere in Form eines K-Drahts, umfasst und wobei das Präparationsinstrument ausgebildet ist zum Verankern des Führungsdrahts im Knochen.

Knochenschrauben werden in vielfältiger Weise in der chirurgischen Orthopädie eingesetzt. Beispielsweise werden sie genutzt, um Stabilisierungselemente von Wirbelsäulenstabilisierungssystemen am Knochen zu verankern und festzulegen. Um einen korrekten Sitz der Knochenschraube im Knochen gewährleisten zu können, wird sehr häufig ein Schraubenloch im Knochen präpariert, in welches die Knochenschraube dann zur Verankerung eingeschraubt wird. Insbesondere zum Heranführen von Knochenschrauben an den Knochen ist es bekannt, einen Führungsdraht zu nutzen, der vorab im Knochen verankert wird. Die Knochenschraube, die in diesem Fall kanüliert ist, also einen durchgängigen Längskanal aufweist, wird zum Platzieren am Knochen über den Führungsdraht geschoben.

Zum Präparieren des Knochens werden üblicherweise mehrere Instrumente eingesetzt. Dies bedeutet für einen Operateur, dass er mehrere Instrumente nacheinander oder auch gleichzeitig Handhaben muss. Ein solcher Eingriff zum Präparieren des Knochens zum Platzieren einer Knochenschraube kann daher aufwendig sein und entsprechend lange dauern.

Aus der US 11,123,113 B2 sind ein Instrument zum Einsetzen von Schrauben sowie ein mit diesem durchführbares Verfahren zum Einsetzen von Schrauben in den Körper eines Patienten bekannt. Das in der genannten Veröffentlichung beschriebene Instrument ist nicht gattungsgemäß ausgebildet und unterscheidet sich von einem Präparationsinstrument zum Präparieren eines Knochens der eingangs beschriebenen Art in seinem konstruktiven Aufbau, und zwar insbesondere dadurch, dass statt eines vom Instrumentenschaft separaten und vollständig trennbaren Führungsdrahts ein Stylet ("stylet 450b") vorgesehen ist. Das Stylet ist an einem Träger ("carrier 450c") dauerhaft festgelegt. Der Träger ist am Instrument über einen Schraubmechanismus in distaler und proximaler Richtung bewegbar, so dass das Stylet von einer gegenüber einem distalen Instrumentenende zurückgezogenen Stellung in eine vorgeschobene Stellung, in welcher ein freies Ende desselben distalseitig über das distale Instrumentenende vorsteht, bringbar ist. Insbesondere kann es distalseitig über ein vorderes Ende einer mit dem distalen Instrumentenende gekoppelten Knochenschraube vorgeschoben werden. Das Instrument kann so am Knochen mit dem Stylet positioniert und die bereits mit dem Instrument gekoppelte Knochenschraube an den Knochen herangeführt und in diesem verankert werden. Das Instrument ist jedoch nicht vorgesehen, den Knochen zu bearbeiten, insbesondere ein Loch für die Knochenschraube zu präparieren. Es ist auch nicht vorgesehen, das Stylet vom Instrument oder vom Träger zu trennen, um es dann, wenn es in einen Knochen eingebracht und dieser mit dem Instrument zum Einsetzen der Knochenschraube präpariert ist, dort allein verankert zu lassen und nach Entfernen des Instruments als Führungselement zum Heranführen einer Knochenschraube an den Knochen zu nutzen. Wie erläutert wird bei dem bekannten Instrument die Knochenschraube mit dem distalen Instrumentenende vorab gekoppelt. Mithin ist also das Stylet nicht vollständig vom Instrumentenschaft trennbar ausgebildet und bildet daher auch keinen separaten Führungsdraht, insbesondere nicht in Form eines K-Drahts.

In der US 2022/0323133 A1 sind eine chirurgische Vorrichtung und ein System zum Einsetzen derselben beschrieben. Auch bei dieser Vorrichtung wird die Knochenschraube vor dem Einbringen eines Führungsdrahts in einen Knochen am distalen Ende der Vorrichtung angeordnet, da die Knochenschraube nicht von proximal über den Führungsdraht geschoben werden kann, da dieser proximalseitig gehalten ist. Die Vorrichtung weist somit strukturell und funktional eine große Ähnlichkeit zu dem aus der US 11,123,113 B2 sind bekannten Instrument auf.

Ein Instrumentarium zum Fassen eines Führungsdrahts für die minimalinvasive Chirurgie ist in der US 2024/0032968 A1 offenbart. Überdies ist aus der DE 10 2018 006 442 A1 ein Instrumenten-Set bekannt.

Es ist daher eine Aufgabe der vorliegenden Erfindung, ein Präparationsinstrument der eingangs beschriebenen Art so zu verbessern, dass eine Präparation des Knochens vor der Verankerung einer Knochenschraube vereinfacht wird.

Diese Aufgabe wird bei einem Präparationsinstrument der eingangs beschriebenen Art erfindungsgemäß dadurch gelöst, dass der Führungsdraht mit dem Präparationsinstrument koppelbar ausgebildet ist, insbesondere zum Führen des Präparationsinstruments beim Präparieren des Knochens, und dass das Präparationsinstrument einen Führungsdrahtbewegungsmechanismus umfasst zum Bewegen des Führungsdrahts relativ zum distalen Ende in distaler und/oder proximaler Richtung.

Ein Präparationsinstrument in der vorgeschlagenen Weise weiterzubilden, ermöglicht es insbesondere, mit dem Präparationsinstrument den Führungsdraht in gewünschter Weise nicht nur am Knochen zu platzieren, sondern ihn mit dem Führungsdrahtbewegungsmechanismus auch in den Knochen einzutreiben. Hierzu bedarf es also nicht zwingend eines Schlaginstruments. Vielmehr kann der Führungsdraht gefühlvoll in distaler Richtung bewegt werden, und zwar mit dem Führungsdrahtbewegungsmechanismus, mit welchem ein distales Ende des Führungsdrahts in distaler Richtung über das distale Ende des Präparationsinstruments hinaus bewegbar ist. Der wie beschrieben im Knochen platzierte Führungsdraht kann insbesondere zum Führen des Präparationsinstruments dienen. Dieses kann relativ zum Führungsdraht in distaler und proximaler Richtung bewegt und dabei geführt werden, insbesondere zum Präparieren des Knochens auf unterschiedliche Arten, wie nachfolgend noch näher erläutert werden wird. Überdies kann der in den Knochen eingebrachte Führungsdraht vom Instrument vollständig getrennt und dann als Führungselement zum Heranführen einer Knochenschraube oder eines mit einer Knochenschraube gekoppelten Instruments genutzt werden, um die Knochenschraube dort, wo der Knochen mit dem Präparationsinstrument präpariert wurde, zu verankern. Der Führungsdraht kann dann nach dem Verankern der Knochenschraube im Knochen wieder entfernt werden. Diese Vorgehensweise ist mit den aus der US 11,123,113 B2 und der US 2022/0323133 A1 bekannten Instrumenten und Vorrichtungen nicht möglich.

Günstig ist es, wenn das Präparationsinstrument einen sich parallel zur Instrumentenlängsachse erstreckenden, das Präparationsinstrument durchsetzenden Längskanal umfasst. Der Längskanal kann insbesondere das Präparationsinstrument vollständig durchsetzen. Der Längskanal kann insbesondere vollständig durchgängig ausgebildet sein, sodass ein entsprechend langer Führungsdraht sowohl distalseitig als auch proximalseitig aus dem Längskanal des Präparationsinstruments vorstehen kann. Diese Ausgestaltung ermöglicht es einem Operateur, insbesondere unmittelbar zu erkennen, ob der Führungsdraht mit dem Präparationsinstrument gekoppelt ist, nämlich dann, wenn das proximale Ende des Führungsdrahts sich relativ zum proximalen Ende des Präparationsinstruments nicht bewegt beziehungsweise nicht mehr bewegen kann. Ferner kann so das Präparationsinstrument bei Bedarf auch noch weiter in distaler Richtung relativ zum Führungsdraht bewegt werden, beispielsweise wenn das Präparationsinstrument vom Führungsdraht entkoppelt wird.

Vorzugsweise erstreckt sich der Längskanal durchgängig vom distalen Ende bis zu einem proximalen Ende des Präparationsinstruments. Wie bereits erläutert kann so einem Operateur insbesondere eine volle Kontrolle über das Präparationsinstrument sowie eine relative Positionierung desselben zum Führungsdraht ermöglicht werden.

Vorteilhaft ist es, wenn das Präparationsinstrument einen distalen und einen proximalen Instrumententeil umfasst, wenn der Führungsdrahtbewegungsmechanismus den distalen und den proximalen Instrumententeil umfasst und wenn der distale und der proximale Instrumententeil relativ zueinander bewegbar, insbesondere parallel zur Instrumentenlängsachse, ausgebildet sind. Eine solche Ausgestaltung ermöglicht es insbesondere, einen Abstand zwischen dem distalen Ende des Präparationsinstruments und einem proximalen Ende desselben zu verändern. Beispielsweise dann, wenn der Führungsdraht mit dem proximalen Instrumententeil koppelbar ist, kann der Führungsdraht relativ zum distalen Instrumententeil in distaler Richtung bewegt werden, und zwar auch dann, wenn der distale Instrumententeil relativ zum Knochen unbeweglich platziert wird. Das Vorschieben und Eintreiben des Führungsdrahts in den Knochen kann so auf einfache Weise realisiert werden. Insbesondere kann der Führungsdrahtbewegungsmechanismus auch eine Bewegung des Führungsdrahts in proximaler Richtung relativ zum distalen Ende des Präparationsinstruments ermöglichen. Die Kopplung des distalen Instrumententeils und des proximalen Instrumententeils kann insbesondere vor der Anwendung oder einem Einsatz des Präparationsinstrumentes erfolgen. Ferner ist die Kopplung auch während eines Einsatzes oder der Anwendung des Präparationsinstruments, also in situ, möglich. Bei der Kopplung in situ kann insbesondere so vorgegangen werden, dass zuerst der distale Instrumententeil am Knochen angesetzt wird. Dann kann eine Kontrolle der Positionierung des distalen Instrumententeils am Knochen mittels bildgebendem Verfahren, zum Beispiel unter Ultraschall- oder Röntgenbeobachtung, durchgeführt werden. Anschließend kann dann der proximale Instrumententeil, insbesondere mit an diesem gekoppeltem Führungsdraht, mit dem am Knochen anliegenden distalen Instrumententeil in Eingriff gebracht werden, insbesondere in diesen eingeführt beziehungsweise eingeschraubt werden.

Auf einfache Weise lässt sich der Führungsdrahtbewegungsmechanismus ausbilden, wenn er eine Bewegungsgewindeverbindung umfasst und wenn der distale und der proximale Instrumententeil über die Bewegungsgewindeverbindung miteinander verschraubbar koppelbar sind. Die Bewegungsgewindeverbindung ermöglicht es insbesondere zum einen, die beiden Instrumententeile miteinander zu koppeln, und zum anderen, die beiden Instrumententeile relativ zueinander zu bewegen, nämlich zu verschrauben. Durch eine Schraubbewegung der Instrumententeile relativ zueinander kann ein Abstand zwischen dem distalen und proximalen Ende des Präparationsinstruments verändert werden, nämlich insbesondere vergrößert beziehungsweise verkleinert.

Der Führungsdrahtbewegungsmechanismus kann insbesondere auf einfache Weise dadurch ausgebildet werden, dass die Bewegungsgewindeverbindung zwei zusammenwirkende Gewindeabschnitte umfasst, welche in Form eines Innengewindeabschnitts und eines zu diesem korrespondierenden Außengewindeabschnitts ausgebildet sind, und wenn der eine der zwei Gewindeabschnitte am distalen Instrumententeil angeordnet oder ausgebildet ist und wenn der andere der zwei Gewindeabschnitte am proximalen Instrumententeil angeordnet oder ausgebildet ist. Durch diese Ausgestaltung lassen sich die zwei Instrumententeile auf einfache Weise durch Verschrauben miteinander koppeln und auch relativ zueinander bewegen, beispielsweise dann, wenn der distale Instrumententeil am Knochen anliegt, kann durch Verschrauben des proximalen Instrumententeils ein proximales Ende desselben in distaler oder proximaler Richtung bewegt werden. Ist insbesondere der Führungsdraht mit dem proximalen Instrumententeil gekoppelt, kann so der Führungsdraht wahlweise in distaler und proximaler Richtung bewegt werden. Insbesondere kann so durch eine Verschraubbewegung der Instrumententeile relativ zueinander der Führungsdraht gefühlvoll in den Knochen eingetrieben werden.

Vorteilhaft ist es, wenn das Präparationsinstrument eine Instrumentensicherungseinrichtung umfasst, welche ausgebildet ist, den distalen und den proximalen Instrumententeil miteinander verbunden zu halten, wenn sie nicht über die Bewegungsgewindeverbindung miteinander gekoppelt sind. So kann insbesondere ein undefiniertes und unerwünschtes Auseinanderfallen der distalen und proximalen Instrumententeile vermieden werden, wenn beispielsweise die zwei zusammenwirkenden Gewindeabschnitte der Bewegungsgewindeverbindung vollständig auseinandergeschraubt und voneinander getrennt sind, also außer Eingriff stehen.

Die Instrumentensicherungseinrichtung kann insbesondere auf einfache Weise ausgebildet werden, wenn sie zwei zusammenwirkende Instrumentensicherungseinrichtungselemente umfasst, die einerseits am distalen Instrumententeil und andererseits am proximalen Instrumententeil angeordnet oder ausgebildet sind und die in einer Verbindungsstellung miteinander in Eingriff stehen, um den distalen und den proximalen Instrumententeil aneinander zu halten.

Günstigerweise sind die zwei zusammenwirkenden Instrumentensicherungseinrichtungselemente in Form eines Sicherungsrings und in Form einer Sicherungsnut ausgebildet, wobei der Sicherungsring in der Verbindungsstellung in die Sicherungsnut eingreift. So lassen sich der distale und der proximale Instrumententeil auch dann verbunden aneinander halten, wenn die Gewindeabschnitt der Bewegungsgewindeverbindung vollständig außer Eingriff stehen.

Günstig ist es, wenn der distale Instrumententeil den Innengewindeabschnitt und wenn der proximale Instrumententeil den Außengewindeabschnitt umfasst. Dies ermöglicht es insbesondere, den distalen Instrumententeil mit einer glatten äußeren, gewindefreien Hülle auszubilden, die ein Operateur beispielsweise gut mit einer Hand ergreifen kann, ohne dass er Gefahr laufen muss, dass ein Außengewindeabschnitt einen von ihm getragenen Handschuh beschädigt oder verletzt.

Vorzugsweise erstreckt sich der Außengewindeabschnitt ausgehend von einem distalen Ende des proximalen Instrumententeils in proximaler Richtung. Dies ermöglicht insbesondere einen kompakten Aufbau des Präparationsinstruments. So wird unmittelbar eine Kopplung der beiden Instrumententeile miteinander ermöglicht, wenn das distale Ende des proximalen Instrumententeils mit dem korrespondierenden Gewindeabschnitt des distalen Instrumententeils in Kontakt tritt.

Gemäß einer weiteren bevorzugten Ausführungsform kann vorgesehen sein, dass der Führungsdrahtbewegungsmechanismus ausgebildet ist in Form eines Vorschubmechanismus zum Vorschieben des Führungsdrahts relativ zum distalen Ende in distaler Richtung. Eine solche Ausgestaltung hat insbesondere den Vorteil, dass der Führungsdraht selbst nicht rotieren muss, wenn er in distaler Richtung bewegt und in den Knochen eingetrieben wird. Ein solcher Vorschubmechanismus kann also insbesondere ausschließlich eine Vorschubbewegung realisieren, ohne eine Rotation des Führungsdrahts beim Bewegen desselben relativ zum distalen Ende des Präparationsinstruments. Dies ist insbesondere auch dann möglich, wenn die beiden Instrumententeile zum Bewegen des Führungsdrahts relativ zueinander verdreht werden, insbesondere durch eine Schraubbewegung.

Ferner ist es vorteilhaft, wenn der Führungsdrahtbewegungsmechanismus eine Mitnahmeeinrichtung umfasst, wenn die Mitnahmeeinrichtung und der Führungsdraht in einer Mitnahmestellung kraft- und/oder formschlüssig bezogen auf die Instrumentenlängsachse in axialer Richtung gekoppelt in Eingriff und in einer Einführstellung außer Eingriff stehen. Mit der Mitnahmeeinrichtung ist es also insbesondere möglich, das Präparationsinstrument mit dem Führungsdraht zu koppeln, und zwar temporär in der Mitnahmestellung. Das Präparationsinstrument kann beispielsweise über den Führungsdraht geschoben oder von diesem abgezogen werden, wenn die Mitnahmeeinrichtung die Einführstellung einnimmt. Die Mitnahmeeinrichtung kann insbesondere ausgebildet sein, um drehfest mit dem Führungsdraht in der Mitnahmestellung gekoppelt zu sein. Die Mitnahmeeinrichtung kann wahlweise am distalen oder am proximalen Instrumententeil angeordnet oder ausgebildet sein.

Vorteilhaft ist es, wenn der Führungsdraht und die Mitnahmeeinrichtung in der Mitnahmestellung bezogen auf die Instrumentenlängsachse relativ zueinander verdrehbar sind. Dies ermöglicht es insbesondere, die Instrumententeile relativ zueinander zu verdrehen, um den Führungsdraht in axialer Richtung zu bewegen, und zwar ohne dass der Führungsdraht dabei mitrotiert. Die Kopplung zwischen der Mitnahmeeinrichtung und dem Führungsdraht führt lediglich zu einer Sicherung in axialer Richtung, also derart, dass der Führungsdraht weder in distaler noch in proximaler Richtung relativ zur Mitnahmeeinrichtung bewegt werden kann, wenn die Mitnahmestellung eingenommen wird.

Der Führungsdrahtbewegungsmechanismus lässt sich auf einfache Weise ausbilden, wenn die Mitnahmeeinrichtung ein Mitnehmerelement umfasst, welches in der Mitnahmestellung mit einem Führungsdrahtmitnahmeglied des Führungsdrahts kraft- und/oder formschlüssig in Eingriff steht und in einer Einführstellung außer Eingriff steht. Zum Überführen der Mitnahmeeinrichtung von der Mitnahmestellung in die Einführstellung und umgekehrt muss dann lediglich das Mitnehmerelement relativ zum Führungsdraht bewegt werden, beispielsweise auf diesen hin zum Überführen in die Mitnahmestellung oder von diesem weg zum Überführen der Mitnahmeeinrichtung in die Einführstellung, in welcher eine Relativbewegung des Führungsdrahts zur Mitnahmeeinrichtung in axialer Richtung, also parallel zur Instrumentenlängsachse möglich ist.

Auf einfache Weise lässt sich die Mitnahmeeinrichtung ausbilden, wenn das Mitnehmerelement in Form eines Mitnehmervorsprungs oder einer Mitnehmerausnehmung ausgebildet ist und wenn das Führungsdrahtmitnahmeglied korrespondierend ausgebildet ist in Form einer Führungsdrahtausnehmung oder eines Führungsdrahtvorsprungs. Beispielsweise kann der Mitnehmervorsprung in der Mitnahmestellung in die Führungsdrahtausnehmung eingreifen oder bei einer alternativen Ausgestaltung die Mitnehmerausnehmung den Führungsdrahtvorsprung in der Mitnahmestellung aufnehmen.

Auf einfache Weise lässt sich die Mitnahmeeinrichtung realisieren, wenn die Führungsdrahtausnehmung in Form einer Nut ausgebildet ist. Insbesondere kann die Nut in Form einer von der Instrumentenlängsachse in radialer Richtung weg weisend geöffneten Ringnut ausgebildet sein. Diese Ausgestaltung ermöglicht es insbesondere, den Führungsdraht und die Mitnahmeeinrichtung einfach und zuverlässig in der Mitnahmestellung zu koppeln, und zwar unabhängig von einer Rotationsstellung des Führungsdrahts bezogen auf die Instrumentenlängsachse.

Vorteilhaft ist es, wenn das Mitnehmerelement in radialer Richtung bezogen auf die Instrumentenlängsachse bewegbar, insbesondere verschiebbar, angeordnet oder ausgebildet ist. So kann beispielsweise ein Anwender durch Bewegen des Mitnehmerelements auf die Instrumentenlängsachse hin das Präparationsinstrument und den Führungsdraht miteinander koppeln, sodass die Mitnahmeeinrichtung die Mitnahmestellung einnimmt, oder durch entsprechende Bewegung in umgekehrter Richtung den Führungsdraht und das Präparationsinstrument entkoppeln, um sie relativ zueinander in axialer Richtung bewegen zu können. Alternativ kann das Mitnehmerelement auch verschwenkbar oder verdrehbar angeordnet beziehungsweise ausgebildet sein.

Für eine einfache Kopplung des Präparationsinstruments und des Führungsdrahts ist es vorteilhaft, wenn das Mitnehmerelement in der Mitnahmestellung mindestens teilweise in den Längskanal hinein vorsteht. So kann es insbesondere in eine Führungsdrahtausnehmung am Führungsdraht in der Mitnahmestellung einfach und zuverlässig eingreifen.

Vorteilhaft ist es, wenn das Mitnehmerelement von der Mitnahmestellung entgegen der Wirkung einer vom Präparationsinstrument umfassten Rückstelleinrichtung in die Einführstellung bewegbar ist. Eine solche Rückstelleinrichtung ermöglicht es insbesondere, das Mitnehmerelement in einer Grundstellung oder einer Grundposition automatisch in der Mitnahmestellung zu halten. Dies hat insbesondere den Vorteil, dass ein Operateur aktiv die Mitnahmeeinrichtung betätigen muss, um das Präparationsinstrument und den Führungsdraht voneinander zu entkoppeln.

Günstigerweise umfasst die Rückstelleinrichtung mindestens ein Rückstellelement. Ein solches Rückstellelement kann insbesondere die erforderliche Kraft ausüben, die überwunden werden muss, um die Mitnahmeeinrichtung von der Mitnahmestellung in die Einführstellung zu überführen.

Auf einfache Weise lässt sich die Rückstelleinrichtung ausbilden, wenn das mindestens eine Rückstellelement in Form einer Feder ausgebildet ist. Insbesondere kann die Feder in Form einer Schraubenfeder oder einer Blattfeder ausgebildet sein. Die Feder kann zudem sowohl als Druck- als auch als Zugfeder realisiert werden, abhängig vom konstruktiven Aufbau der Mitnahmeeinrichtung.

Um das Präparationsinstrument möglichst kompakt ausbilden zu können, ist es günstig, wenn die Rückstelleinrichtung in radialer Richtung auf die Instrumentenlängsachse hin wirkend angeordnet oder ausgebildet ist. So kann die von der Rückstelleinrichtung aufzubringende Kraft unmittelbar in der Richtung wirken, in der sie benötigt wird.

Vorzugsweise nimmt die Mitnahmeeinrichtung in einer Grundstellung die Mitnahmestellung ein. Wie bereits erläutert hat dies den Vorteil, dass ein Operateur die Mitnahmeeinrichtung aktiv betätigen muss, um das Präparationsinstrument und den Führungsdraht voneinander zu entkoppeln.

Um ein unbeabsichtigtes Entkoppeln des Präparationsinstruments und des Führungsdrahts voneinander zu vermeiden, ist es vorteilhaft, wenn das Mitnehmerelement in der Mitnahmestellung unter Vorspannung gehalten ist.

Um eine einfache und schnelle Kopplung des Präparationsinstruments und des Führungsdrahts in der Mitnahmestellung zu ermöglichen, ist es günstig, wenn die Mitnahmeeinrichtung in Form einer Rast- oder Schnappverbindungseinrichtung ausgebildet ist. Die zusammenwirkenden Komponenten, nämlich das Mitnehmerelement einerseits und das Führungsdrahtmitnahmeglied andererseits, können bei einer solchen Ausgestaltung in Form von Rast- oder Schnappgliedern ausgebildet sein, die korrespondierend zueinander ausgebildet sind.

Grundsätzlich ist es denkbar, die Mitnahmeeinrichtung am distalen Instrumententeil anzuordnen oder auszubilden. Günstig ist es jedoch, wenn die Mitnahmeeinrichtung am proximalen Instrumententeil angeordnet oder ausgebildet ist. Dies vereinfacht eine Handhabung für einen Operateur, da beispielsweise der distale Instrumententeil ganz oder teilweise in den Körper eines Patienten eingeführt werden muss, um den Knochen in gewünschter Weise zu präparieren. Durch die vorgeschlagene Anordnung der Mitnahmeeinrichtung kann so erreicht werden, dass die Mitnahmeeinrichtung für den Operateur in jedem Fall zuverlässig erreichbar und betätigbar bleibt.

Vorteilhaft ist es, wenn die Mitnahmeeinrichtung ein Betätigungsglied umfasst, welches derart angeordnet oder ausgebildet ist, dass durch Einwirkung einer Betätigungskraft auf das Betätigungsglied die Mitnahmeeinrichtung von der Mitnahmestellung in die Einführstellung überführbar ist. Ein solches Betätigungsglied kann von einem Operateur bei Bedarf betätigt werden, um das Präparationsinstrument und den Führungsdraht voneinander zu entkoppeln. Beispielsweise kann das Betätigungsglied in Form eines Betätigungsknopfes oder eines Betätigungshebels ausgebildet sein, welcher an einem der beiden Instrumententeile angeordnet oder ausgebildet ist.

Vorzugsweise umfasst die Mitnahmeeinrichtung ein Gehäuse und steht das Betätigungsglied zumindest in der Mitnahmestellung aus dem Gehäuse vor. Auf diese Weise kann ein Operateur bei Bedarf das Betätigungsglied erkennen und betätigen. Indem das Betätigungsglied aus dem Gehäuse vorsteht, kann ein Operateur zudem erkennen, dass die Mitnahmeeinrichtung die Mitnahmestellung einnimmt.

Vorteilhafterweise sind das Betätigungsglied und das Mitnehmerelement einstückig, insbesondere monolithisch ausgebildet. So lässt sich die Mitnahmeeinrichtung einfach ausbilden. Zudem kann ein Operateur durch Krafteinwirkung auf das Betätigungsglied auch das Mitnehmerelement unmittelbar mit dieser Betätigungskraft beaufschlagen und in gewünschter Weise bewegen.

Wie bereits eingangs erwähnt, soll das Präparationsinstrument das Präparieren des Knochens auf einfache Weise ermöglichen. Insbesondere soll ein Operateur in die Lage versetzt werden, sämtliche Präparationsschritte vor der Verankerung einer Knochenschraube am Knochen mit nur einem einzigen Instrument durchzuführen. Daher ist es vorteilhaft, wenn das Präparationsinstrument mindestens ein Werkzeugelement zum Präparieren des Knochens umfasst. Idealerweise ist das Präparationsinstrument also als sogenanntes "All-in-one"-Instrument ausgebildet, also ein Instrument, mit dem alle Präparationsschritte durchgeführt werden können. Dies hat einen großen Vorteil, da ein Operateur dann lediglich ein einziges Instrument handhaben muss, um den Knochen in gewünschter Weise zu präparieren. Ein Wechsel zwischen unterschiedlichen Instrumenten ist dann nicht erforderlich. Insbesondere kann das Präparationsinstrument mehrere Werkzeugelemente umfassen, mit denen der Knochen in unterschiedlicher Weise präpariert werden kann. Dies wird nachfolgend noch im Einzelnen erläutert.

Um den Knochen auf einfache Weise präparieren zu können, ist es vorteilhaft, wenn das mindestens eine Werkzeugelement am distalen Instrumententeil in distaler Richtung weisend angeordnet oder ausgebildet ist. So kann es unmittelbar mit dem zu präparierenden Knochen in Kontakt gebracht werden.

Vorteilhaft ist es, wenn das mindestens eine Werkzeugelement in Form eines Perforationswerkzeugs zum Perforieren der Kortikalis des Knochens ausgebildet ist. Als Kortikalis wird die Knochenschicht bezeichnet, die das schwammähnliche Innengewebe, die Spongiosa, des Knochens außen umgibt. Die Kortikalis ist besonders hart, sodass es den Knochen vor Druck-, Biege- und Drehkräften schützen kann. Verständlicherweise ist das Einführen eines Führungsdrahts durch die Kortikalis hindurch schwierig. Das Perforationswerkzeug ermöglicht es insbesondere, die Kortikalis auf einfache Weise zu perforieren, sodass ein Zugang zur Spongiosa des Knochens ermöglicht wird, in den hinein sich der Führungsdraht leichter eintreiben lässt.

Um die Kortikalis einfach und zuverlässig perforieren zu können, ist es günstig, wenn das Perforationswerkzeug mindestens eine Spitze, insbesondere eine Mehrzahl von Spitzen, umfasst und wenn die mindestens eine Spitze bezogen auf die Längsachse in radialer Richtung versetzt angeordnet oder ausgebildet ist. Die mindestens eine Spitze, es können eben auch zwei, drei oder mehr Spitzen vorgesehen sein, lässt sich auf einfache Weise in die Kortikalis eintreiben, beispielsweise durch Einschlagen mithilfe eines Schlagwerkzeugs, beispielsweise eines Schlaghammers. Die Positionierung der Spitze seitlich versetzt zur Instrumentenlängsachse hat insbesondere den Vorteil, dass der Führungsdraht trotzdem koaxial zur Längsachse des Längskanals bewegt werden kann, wenn das Perforationswerkzeug in die Kortikalis eingetrieben ist.

Vorzugsweise umfasst das Perforationswerkzeug eine Mehrzahl von Spitzen, welche die Instrumentenlängsachse umgebend angeordnet oder ausgebildet sind. Auf diese Weise lässt sich ein kronenartiges Ende des Präparationsinstruments, insbesondere des distalen Instrumententeils, ausbilden. Beispielsweise können drei, vier, fünf oder mehr Spitzen vorgesehen sein, die den Längskanal begrenzend das distale Ende des Präparationsinstruments definieren. Mit einer solchen Mehrzahl von Spitzen lässt sich bei entsprechender Gestaltung, beispielsweise schneidenartig, ein scheibenförmiges Kortikaliselement ausstanzen, sodass durch diese Öffnung in der Kortikalis das distale Ende des Führungsdrahts in den Knochen hineinbewegt werden kann.

Ferner ist es günstig, wenn das mindestens eine Werkzeugelement in Form eines Bohrwerkzeugs zum Bohren eines Schraubenlochs im Knochen ausgebildet ist. Mit diesem Bohrwerkzeug kann insbesondere auf einfache Weise ein Schraubenloch im Knochen ausgebildet werden. Beispielsweise kann hierfür ein Operateur den proximalen Instrumententeil festhalten und den distalen Instrumententeil relativ zum proximalen Instrumententeil in distaler Richtung schraubend bewegen, um dadurch das Schraubenloch von Hand in den Knochen zu bohren.

Günstig ist es, wenn das Bohrwerkzeug proximalseitig des Perforationswerkzeug angeordnet oder ausgebildet ist. Diese Ausgestaltung ermöglicht es einem Operateur insbesondere, zunächst mit dem Perforationswerkzeug die Kortikalis zu perforieren, mit dem Präparationsinstrument den Führungsdraht in den Knochen einzuschieben und in einem nächsten Schritt mit dem Bohrwerkzeug das Schraubenloch auszubilden. Dabei muss er kein Instrument wechsein. Er kann alles mit dem einen Präparationsinstrument durchführen.

Ferner kann es vorteilhaft sein, wenn mindestens eine Werkzeugelement in Form eines Gewindeschneidwerkzeugs zum Schneiden eines Gewindes in das Schraubenloch im Knochen ausgebildet ist. Ein solches Werkzeugelement vorzusehen ermöglicht es einem Operateur insbesondere, den Knochen weiter zu präparieren, um so das Einschrauben einer Knochenschraube in den Knochen in definierter Weise vorzugeben. Das Ausbilden eines Gewindes im Schraubenloch ist insbesondere dann vorteilhaft, wenn Knochenschrauben eingesetzt werden, die kein selbstschneidendes Gewinde umfassen.

Vorteilhafterweise ist das Gewindeschneidwerkzeug proximalseitig des Bohrwerkzeugs angeordnet oder ausgebildet. Eine solche Ausgestaltung ermöglicht es insbesondere, das Schraubenloch zu bohren und in einem Arbeitsschritt beim Bohren gleichzeitig ein Gewinde in das Schraubenloch zu schneiden. Dabei ist es nicht zwingend erforderlich, dass sich das Gewinde über die gesamte Länge des Schraubenlochs erstreckt. Insbesondere kann sich das Gewindeschneidwerkzeug unmittelbar an das Bohrwerkzeug proximalseitig anschließen.

Ferner ist es günstig, wenn das Bohrwerkzeug eine Bohrwerkzeuglänge aufweist, wenn das Gewindeschneidwerkzeug eine Gewindeschneidwerkzeuglänge aufweist und wenn ein Verhältnis von Gewindeschneidwerkzeuglänge und Bohrwerkzeuglänge höchstens etwa 1 beträgt. Mit anderen Worten bedeutet dies, dass das Gewindeschneidwerkzeug höchstens so lang ist wie das Bohrwerkzeug. Oder anders ausgedrückt ist die Gewindeschneidwerkzeuglänge nicht größer als die Bohrwerkzeuglänge.

Günstig ist es, wenn der distale Instrumententeil einen distalen Schaft umfasst und wenn sich das mindestens eine Werkzeugelement in distaler Richtung weisend vom distalen Schaft weg erstreckt. Beispielsweise kann der Schaft in Form einer langgestreckten zylindrischen Hülse ausgebildet sein mit einer glatten Außenfläche, sodass ein Operateur den distalen Instrumententeil einfach und zuverlässig handhaben kann. Insbesondere kann sich das mindestens eine Werkzeugelement koaxial zur Instrumentenlängsachse vom distalen Schaft weg erstrecken. Zudem kann sich der Längskanal koaxial zur Instrumentenlängsachse durch das mindestens eine Werkzeugelement hindurch erstrecken.

Vorzugsweise ist ein Außendurchmesser des distalen Schafts größer ist als ein vom mindestens einen Werkzeugelement definierter Werkzeugaußendurchmesser. So lässt sich ein kompaktes Präparationsinstrument ausbilden.

Für eine einfache Kopplung der Instrumententeile miteinander ist es vorteilhaft, wenn der Innengewindeabschnitt im Bereich des distalen Schafts angeordnet oder ausgebildet ist. Hier ist insbesondere genügend Raum verfügbar, um den proximalen Instrumententeil mit einem Außengewindeabschnitt mit dem Innengewindeabschnitt des distalen Schafts zu verschrauben.

Um das Präparationsinstrument noch universeller einsetzen zu können ist es günstig, wenn es eine Einführtiefenmesseinrichtung umfasst zum Bestimmen eines Abstands des distalen Endes des aus dem distalen Ende des Präparationsinstruments vorstehenden Führungsdrahts vom distalen Ende des Präparationsinstruments. Auf diese Weise lässt sich insbesondere messen, welche Schraubenlänge der Knochenschraube zum Verankern im Knochen gewählt werden kann beziehungsweise höchstens gewählt werden kann. Insbesondere kann der Führungsdraht unter Ultraschall- oder Röntgenkontrolle in den Knochen vorgetrieben werden, um so sicherstellen zu können, dass keine Nerven oder Blutgefäße des Patienten verletzt werden. Dies ist insbesondere von größter Bedeutung beim Platzieren von Knochenschrauben in Form von Pedikelschrauben an Wirbelkörpern oder Teilen derselben, insbesondere Pedikeln, beim Versorgen eines Patienten mit einem Wirbelsäulenstabilisierungssystem. So lässt sich das Perforationsinstrument wie bereits beschrieben mit seinem distalen Ende am Knochen ansetzen und dann der Führungsdraht in distaler Richtung über das distale Ende des Präparationsinstruments hinaus in den Knochen vorschieben. Erfolgt dies wie erläutert unter Kontrolle, dann kann durch die Vorschublänge des Führungsdrahts, die direkt am Instrument ablesbar ist, die Länge der maximal verankerbaren Knochenschraube bestimmt werden. Der Operateur kann eine Knochenschraube mit dieser Länge wählen, um sie später, nach abgeschlossener Präparation des Knochens, in diesem zu verankern.

Die Einführtiefenmesseinrichtung lässt sich auf einfache Weise ausbilden, wenn sie eine Mehrzahl von Markierungselementen umfasst und wenn die Markierungselemente proximalseitig des vom proximalen Instrumententeil umfassten Gewindeabschnitts am proximalen Instrumententeil angeordnet oder ausgebildet sind. Die Markierungselemente sind dann insbesondere sichtbar, solange der proximale Instrumententeil nicht so weit in den distalen Instrumententeil hinein verschraubt ist, dass der Gewindeabschnitt am proximalen Instrumententeil nicht mehr sichtbar ist. Die Zahl der sichtbaren Markierungselemente verringert sich weiter, wenn der proximale Instrumententeil weiter in distaler Richtung mit dem distalen Instrumententeil verschraubt wird. Durch entsprechende Beschriftung der Markierelemente kann ein Operateur dann unmittelbar ablesen, wie weit das distale Ende des Führungsdrahts über das distale Ende des Präparationsinstruments hinaus vorbewegt wurde. Die Markierungselemente können insbesondere in Form äquidistanter Ringmarkierungen mit Beschriftung am proximalen Instrumententeil angeordnet oder ausgebildet sein. Sie können insbesondere durch Gravieren oder Laserbeschriften ausgebildet sein.

Gemäß einer weiteren bevorzugten Ausführungsform kann vorgesehen sein, dass das Präparationsinstrument einen distalen Instrumentengriff zum Drehen des distalen Instrumententeils um die Instrumentenlängsachse umfasst. Mit dem distalen Instrumentengriff kann also ein Operateur den distalen Instrumententeil nicht nur handhaben, sondern auch um die Instrumentenlängsachse rotieren. Beispielsweise kann er bei einer solchen Drehbewegung den proximalen Instrumententeil festhalten. Bei einer solchen Drehbewegung des distalen Instrumententeil kann er dann beispielsweise mit den oben beschriebenen Werkzeugelementen ein Schraubenloch bohren und optional auch mit einem Gewinde versehen.

Für eine einfache und zuverlässige Handhabbarkeit des Präparationsinstruments ist es günstig, wenn der distale Instrumentengriff zwei Griffarme umfasst, welche in radialer Richtung und diametral voneinander weg weisen. Unabhängig von einer Drehstellung des distalen Instrumentengriffs ist dann mindestens einer der zwei Griffarme für einen Operateur gut sichtbar und leicht ergreifbar.

Vorzugsweise ist der distale Instrumentengriff mit dem distalen Instrumententeil lösbar verbindbar ausgebildet. Auf diese Weise lässt sich das Präparationsinstrument zum Reinigen zerlegen und kann zudem kompakt aufbewahrt werden.

Günstig ist es, wenn der distale Instrumentengriff mit dem distalen Instrumententeil in einer Verbindungsstellung bezogen auf die Instrumentenlängsachse drehfest gekoppelt ist. Diese Ausgestaltung hat insbesondere den Vorteil, dass ein Operateur durch Krafteinwirkung auf den Instrumentengriff den distalen Instrumententeil in gewünschter Weise um die Instrumentenlängsachse verdrehen kann, und zwar sowohl im Uhrzeiger- als auch im Gegenuhrzeigersinn.

Um den distalen Instrumentengriff auf einfache Weise mit dem distalen Instrumententeil koppeln zu können, ist es vorteilhaft, wenn das Präparationsinstrument eine Verbindungseinrichtung zum kraft- und/oder formschlüssigen Koppeln des distalen Instrumentengriffs und des distalen Instrumententeils in der Verbindungsstellung umfasst.

Günstig ist es, wenn die Verbindungseinrichtung einen distalen Anschlag umfasst zum Begrenzen einer Bewegung des distalen Instrumentengriffs relativ zum distalen Instrumententeil in der Verbindungsstellung in distaler Richtung. Ein solcher distaler Anschlag hat zudem den Vorteil, dass ein Operateur den Instrumentengriff in distaler Richtung drücken und damit auch den distalen Instrumententeil in distaler Richtung drücken kann. Beispielsweise lässt sich so auch mit dem oben beschriebenen Perforationswerkzeug ohne Einsatz eines Schlagwerkzeugs die Kortikalis gegebenenfalls perforieren.

Die Verbindungseinrichtung lässt sich auf einfache Weise ausbilden, wenn der distale Anschlag eine sich quer, insbesondere senkrecht, zur Instrumentenlängsachse erstreckende, in proximaler Richtung weisende distale Anschlagfläche umfasst, an welcher der distale Instrumentengriff in der Verbindungsstellung anliegt.

Günstig ist es, wenn die Verbindungseinrichtung erste und zweite, zusammenwirkende Verbindungselemente umfasst, welche einerseits am distalen Instrumententeil und andererseits am distalen Instrumentengriff angeordnet oder ausgebildet sind, und wenn die ersten und zweiten Verbindungselemente in der Verbindungsstellung kraft- und/oder formschlüssig in Eingriff stehen und in einer Reinigungsstellung außer Eingriff stehen. Eine derart ausgestaltete Verbindungseinrichtung ermöglicht das einfache Koppeln des distalen Instrumentengriffs mit dem distalen Instrumententeil in der Verbindungsstellung.

Vorteilhaft ist es, wenn eines der zusammenwirkenden Verbindungselemente in Form einer Verzahnung ausgebildet ist, insbesondere mit einer Mehrzahl von Zähnen, und wenn eines der Verbindungselemente in Form mindestens eines in der Verbindungsstellung in die Verzahnung eingreifenden Verbindungsvorsprungs ausgebildet ist. Insbesondere kann so beim Eingreifen des mindestens einen Verbindungsvorsprungs in die Verzahnung eine Kopplung, insbesondere eine drehfeste Kopplung, realisiert werden.

Um beispielsweise den distalen Instrumentengriff in Form eines Ratschengriffs ausbilden zu können, also insbesondere um die Stellung der Griffarme zu verändern, ohne dabei eine Drehstellung des distalen Instrumententeils zu verändern, ist es vorteilhaft, wenn die ersten und zweiten Verbindungselemente in einer Drehstellung relativ zueinander um die Instrumentenlängsachse verdrehbar sind.

Günstig ist es, wenn der mindestens eine Verbindungsvorsprung in der Drehstellung in radialer Richtung bezogen auf die Instrumentenlängsachse auslenkbar angeordnet oder ausgebildet ist. Auf diese Weise kann der mindestens eine Verbindungsvorsprung mit der Verzahnung außer Eingriff gebracht werden und so eine Verdrehung des distalen Instrumentengriffs entweder im Uhrzeiger oder im Gegenuhrzeigersinn relativ zum distalen Instrumententeil ermöglicht werden.

Eine solche ratschengriffartige Ausgestaltung lässt sich insbesondere auf einfache Weise realisieren, wenn der mindestens eine Verbindungsvorsprung an einem freien Ende eines Federelements, insbesondere eines Blattfederelements, angeordnet oder ausgebildet ist.

Günstig ist es, wenn die Verbindungseinrichtung einen Verbindungsring umfasst, welcher die in radialer Richtung weisende Verzahnung umgibt und aufweitbar ausgebildet ist. Ein solcher Verbindungsring kann beispielsweise eine Verzahnung am distalen Instrumententeil klemmend umgeben und in der Verbindungsstellung zusätzlich eine kraftschlüssige Verbindung herstellen.

Vorteilhaft ist es, wenn der Verbindungsring einen sich in Umfangsrichtung bezogen auf die Instrumentenlängsachse erstreckenden Aufweitspalt umfasst, wenn der eine Griffarm des distalen Instrumentengriffs über zwei mit jeweils einem von zwei freien, aufeinander zu weisenden Enden des Verbindungsrings verbundenen Griffarmstegen verbunden ist. Die aufeinander zu weisenden Enden des Verbindungsrings definieren den Aufweitspalt und können zum Aufweiten desselben etwas voneinander weg oder zum Verringern desselben aufeinander zu bewegt werden. Die Griffarmstege ermöglichen insbesondere einerseits eine zuverlässige Verbindung des einen Griffarms mit dem Verbindungsring im Bereich des Aufweitspalts und andererseits auch ein Aufweiten des Aufweitspalts bei Bedarf.

Vorzugsweise verlaufen die zwei Griffarmstege parallel zueinander. Dies lässt sich auf einfache Weise realisieren. Insbesondere können sie in Richtung des Griffarms, also parallel zu einer radialen Richtung bezogen auf die Instrumentenlängsachse, verlaufen.

Günstigerweise ist das Federelement zwischen den beiden Griffarmstegen angeordnet oder ausgebildet. So lässt sich das Federelement durch die beiden Griffarmstege geschützt anordnen beziehungsweise ausbilden.

Um eine optimale Einleitung von Drehmomenten auf den distalen Instrumententeil ermöglichen zu können, ist es vorteilhaft, wenn der eine Griffarm am Verbindungsring dem Aufweitspalt bezogen auf die Instrumentenlängsachse diametral gegenüberliegend angeordnet ist.

Ferner ist es günstig, wenn die Verbindungseinrichtung eine Sicherungseinrichtung umfasst zum Sichern der ineinandergreifenden ersten und zweiten Verbindungselemente in der Verbindungsstellung. Insbesondere kann so eine Sicherung dahingehend erreicht werden, dass der Instrumentengriff lediglich in einer Richtung, also beispielsweise entgegen oder im Uhrzeigersinn, relativ zum distalen Instrumententeil verdreht werden kann, in entgegengesetzter Richtung jedoch nicht.

Die Sicherungseinrichtung lässt sich auf einfache Weise ausbilden, wenn sie ein Sicherungselement umfasst, welches am distalen Instrumentengriff oder am distalen Instrumententeil von einer Entsicherungsstellung, in welcher der distale Instrumentengriff und der distale Instrumententeil relativ zueinander in einer Einschraubdrehrichtung bezogen auf die Instrumentenlängsachse verdrehbar sind, in eine Sicherungsstellung bewegbar ist, in welcher der distale Instrumentengriff und der distale Instrumententeil relativ zueinander in der Einschraubdrehrichtung bezogen auf die Instrumentenlängsachse unverdrehbar sind. Die Einschraubdrehrichtung ist insbesondere diejenige Drehrichtung, beispielsweise Uhrzeigersinn oder Gegenuhrzeigersinn, in welcher das Einleiten eines Drehmoments auf den distalen Instrumentengriff zu einer Drehung des distalen Instrumententeils in der Einschraubdrehrichtung führt.

Das Präparationsinstrument lässt sich für einen Operateur insbesondere einfach und kompakt handhabbar ausbilden, wenn das Sicherungselement in Form einer Sicherungshülse ausgebildet ist, welche auf einem Griffarm des distalen Instrumentengriffs in radialer Richtung bezogen auf die Instrumentenlängsachse, insbesondere auf diese hin und von dieser weg, bewegbar, insbesondere verschiebbar, angeordnet oder ausgebildet ist. Insbesondere kann sie auf demjenigen Griffarm bewegbar angeordnet oder ausgebildet sein, welcher die zwei Griffarmstege umfasst.

Vorzugsweise ist das Sicherungselement ausgebildet zum Verhindern eines Aufweitens des Verbindungsrings in der Sicherungsstellung. Es dient also insbesondere dem Zweck, zu verhindern, dass sich der Aufweitspalt vergrößert.

Vorteilhaft ist es, wenn das Sicherungselement die Griffarmstege umschließt und in der Sicherungsstellung eine der Instrumentenlängsachse maximal angenäherte Position und in der Entsicherungsstellung eine von der Instrumentenlängsachse maximal entfernte Position einnimmt. Ein Operateur kann so insbesondere auch aus der Stellung des Sicherungselements direkt erkennen, ob die Sicherungseinrichtung die Sicherungsstellung oder die Entsicherungsstellung einnimmt.

Ferner kann es günstig sein, wenn das Präparationsinstrument einen proximalen Instrumentengriff umfasst zum Drehen des proximalen Instrumententeils um die Instrumentenlängsachse. Auf diese Weise kann ein Operateur insbesondere in definierter und gefühlvoller Weise ein Drehmoment in den proximalen Instrumententeil einleiten. Beispielsweise kann er im Zusammenspiel mit der Mitnahmeeinrichtung durch Drehen des proximalen Instrumententeils relativ zum distalen Instrumententeil den Führungsdraht in distaler Richtung oder proximaler Richtung gefühlvoll bewegen. Für eine einfache Handhabung ist es günstig, wenn der proximale Instrumentengriff in Form eines Ratschengriffs ausgebildet ist. Dieser kann insbesondere umschaltbar ausgebildet sein, so dass er in zwei unterschiedlichen Drehrichtungen genutzt werden kann.

Vorteilhaft ist es, wenn der proximale Instrumentengriff mit dem proximalen Instrumententeil in einer Kopplungsstellung bezogen auf die Instrumentenlängsachse drehfest gekoppelt ist. So kann ein Drehmoment, welches auf den proximalen Instrumentengriff einwirkt, unmittelbar auf den proximalen Instrumententeil übertragen werden.

Günstig ist es, wenn das Präparationsinstrument eine Kopplungseinrichtung umfasst zum kraft- und/oder formschlüssigen Koppeln des proximalen Instrumentengriffs und des proximalen Instrumententeils in der Kopplungsstellung. Diese Ausgestaltung ermöglicht es insbesondere, den proximalen Instrumentengriff bei Bedarf vom Präparationsinstrument abzunehmen, beispielsweise zu Reinigungszwecken oder zum Lagern des Präparationsinstruments.

Die Kopplungseinrichtung lässt sich insbesondere auf einfache Weise ausbilden, wenn sie erste und zweite zusammenwirkende Kopplungselemente umfasst, welche einerseits am proximalen Instrumententeil und andererseits am proximalen Instrumentengriff angeordnet oder ausgebildet sind, und wenn die ersten und zweiten Kopplungselemente in der Kopplungsstellung kraft- und/oder formschlüssig in Eingriff stehen und in einer Entkopplungsstellung außer Eingriff stehen.

Günstig ist es, wenn die Kopplungseinrichtung einen proximalen Anschlag umfasst zum Begrenzen einer Bewegung des proximalen Instrumentengriffs relativ zum proximalen Instrumententeil in der Kopplungsstellung in distaler Richtung. Dies ermöglicht es einem Operateur, auf den proximalen Instrumentengriff eine in distaler Richtung weisende Kraft auszuüben, die dann auch unmittelbar auf den proximalen Instrumententeil übertragen werden kann.

Auf einfache Weise lässt sich die Kopplungseinrichtung ausbilden, wenn der proximale Anschlag eine sich quer, insbesondere senkrecht, zur Instrumentenlängsachse erstreckende, in proximaler Richtung weisende proximale Anschlagfläche umfasst, an welcher der proximale Instrumentengriff in der Kopplungsstellung anliegt.

Für eine kompakte Ausbildung des Präparationsinstruments ist es vorteilhaft, wenn die Mitnahmeeinrichtung die proximale Anschlagfläche definiert oder umfasst.

Günstig ist es, wenn das eine Kopplungselement in Form eines Kopplungsvorsprungs und das andere Kopplungselement in Form einer zum Kopplungsvorsprung korrespondierenden Kopplungsausnehmung ausgebildet ist.

Auf einfache Weise lässt sich der Kopplungsvorsprung in Form eines Außenvielrund oder Außenmehrkant ausbilden. Er kann dann auf einfache Weise mit einer Kopplungsaunehmung in Form eines Innenvielrund oder Innenmehrkant formschlüssig in Eingriff gebracht werden.

Vorzugsweise ist eines der zusammenwirkenden Kopplungselemente im Bereich eines proximalen Endes des proximalen Instrumententeils angeordnet oder ausgebildet. So kann der mit dem Präparationsinstrument gekoppelte proximale Instrumentengriff in der Kopplungsstellung ein proximales Ende des Präparationsinstruments definieren.

Um eine definierte Führung des Führungsdrahts durch das Präparationsinstrument zu ermöglichen, insbesondere ohne diesen zu verformen, nämlich zu verbiegen oder dergleichen, ist es günstig, wenn ein Innendurchmesser des Längskanals im Bereich des proximalen Instrumententeils einem Außendurchmesser des Führungsdrahts entspricht. Insbesondere kann hier ein geringes Spiel vorgesehen sein, um Fertigungstoleranzen auszugleichen.

Ferner ist es günstig, wenn ein Innendurchmesser des Längskanals im Bereich des mindestens einen Werkzeugelements einem Außendurchmesser des Führungsdrahts entspricht. So kann der Führungsdraht auch im distalen Endbereich des Präparationsinstruments, nämlich im Bereich des mindestens einen Werkzeugelements, sicher und definiert geführt werden.

Ferner wird ein Verfahren zum Präparieren eines künstlichen Knochenmodels oder eines menschlichen oder tierischen Knochen vor der Verankerung einer Knochenschraube, insbesondere einer Pedikelschraube, vorgeschlagen, bei welchem Verfahren ein Schraubenloch im Knochenmodell oder im Knochen präpariert wird. Zum Präparieren wird vorgeschlagen, ein Präparationsinstrument zu verwenden. Insbesondere kann es sich dabei um ein Präparationsinstrument handeln, wie es oben in Form bevorzugter Ausführungsformen beschrieben wurde.

Die vorstehende Beschreibung umfasst somit insbesondere die nachfolgend in Form durchnummerierter Sätze definierten Ausführungsformen von Präparationsinstrumenten:
1. Präparationsinstrument (10) zum Präparieren eines Knochens (12) vor der Verankerung einer Knochenschraube (14), insbesondere einer Pedikelschraube, im Knochen (12), wobei das Präparationsinstrument (10) einen eine Instrumentenlängsachse (18) definierenden Instrumentenschaft (16), ein distales Ende (20) und einen vom Instrumentenschaft (16) separaten und vollständig trennbaren Führungsdraht (22), insbesondere in Form eines K-Drahts, umfasst und wobei das Präparationsinstrument (10) ausgebildet ist zum Verankern des Führungsdrahts (22) im Knochen (12), dadurch gekennzeichnet, dass der Führungsdraht (22) mit dem Präparationsinstrument (10) koppelbar ausgebildet ist, insbesondere zum Führen des Präparationsinstruments (10) beim Präparieren des Knochens (12), und dass das Präparationsinstrument (10) einen Führungsdrahtbewegungsmechanismus (184) umfasst zum Bewegen des Führungsdrahts (22) relativ zum distalen Ende (20) in distaler und/oder proximaler Richtung.
2. Präparationsinstrument nach Satz 1, dadurch gekennzeichnet, dass das Präparationsinstrument (10) einen sich parallel zur Instrumentenlängsachse (18) erstreckenden, das Präparationsinstrument (10) durchsetzenden Längskanal (24) umfasst.
3. Präparationsinstrument nach Satz 2, dadurch gekennzeichnet, dass sich der Längskanal (24) durchgängig vom distalen Ende (20) bis zu einem proximalen Ende (26) des Präparationsinstruments (10) erstreckt.
4. Präparationsinstrument nach einem der voranstehenden Sätze, dadurch gekennzeichnet, dass das Präparationsinstrument (10) einen distalen und einen proximalen Instrumententeil (28, 30) umfasst, dass der Führungsdrahtbewegungsmechanismus (184) den distalen und den proximalen Instrumententeil (28, 30) umfasst und dass der distale und der proximale Instrumententeil (28, 30) relativ zueinander bewegbar, insbesondere parallel zur Instrumentenlängsachse (18), ausgebildet sind.
5. Präparationsinstrument nach Satz 4, dadurch gekennzeichnet, dass der Führungsdrahtbewegungsmechanismus (184) eine Bewegungsgewindeverbindung (32) umfasst und dass der distale und der proximale Instrumententeil (28, 30) über die Bewegungsgewindeverbindung (32) miteinander verschraubbar koppelbar sind.
6. Präparationsinstrument nach Satz 5, dadurch gekennzeichnet, dass die Bewegungsgewindeverbindung (32) zwei zusammenwirkende Gewindeabschnitte (112, 114) umfasst, welche in Form eines Innengewindeabschnitts (116) und eines zu diesem korrespondierenden Außengewindeabschnitts (118) ausgebildet sind, und dass der eine der zwei Gewindeabschnitte (112, 114) am distalen Instrumententeil (28) angeordnet oder ausgebildet ist und dass der andere der zwei Gewindeabschnitte (112, 114) am proximalen Instrumententeil (30) angeordnet oder ausgebildet ist.
7. Präparationsinstrument nach Satz 6, dadurch gekennzeichnet, dass der distale Instrumententeil (28) den Innengewindeabschnitt (116) und dass der proximale Instrumententeil (30) den Außengewindeabschnitt (118) umfasst.
8. Präparationsinstrument nach Satz 6 oder 7, dadurch gekennzeichnet, dass sich der Außengewindeabschnitt (118) ausgehend von einem distalen Ende (120) des proximalen Instrumententeils (30) in proximaler Richtung erstreckt.
9. Präparationsinstrument nach einem der voranstehenden Sätze, dadurch gekennzeichnet, dass der Führungsdrahtbewegungsmechanismus (184) ausgebildet ist in Form eines Vorschubmechanismus (186) zum Verschieben des Führungsdrahts (22) relativ zum distalen Ende (20) in distaler Richtung.
10. Präparationsinstrument nach einem der voranstehenden Sätze, dadurch gekennzeichnet, dass der Führungsdrahtbewegungsmechanismus (184) eine Mitnahmeeinrichtung (132) umfasst, dass die Mitnahmeeinrichtung (132) und der Führungsdraht (22) in einer Mitnahmestellung kraft- und/oder formschlüssig bezogen auf die Instrumentenlängsachse (18) in axialer Richtung gekoppelt in Eingriff und in einer Einführstellung außer Eingriff stehen.
11. Präparationsinstrument nach Satz 10, dadurch gekennzeichnet, dass der Führungsdraht (22) und die Mitnahmeeinrichtung (132) in der Mitnahmestellung bezogen auf die Instrumentenlängsachse (18) relativ zueinander verdrehbar sind.
12. Präparationsinstrument nach Satz 10 oder 11, dadurch gekennzeichnet, dass die Mitnahmeeinrichtung (132) ein Mitnehmerelement (166) umfasst, welches in der Mitnahmestellung mit einem Führungsdrahtmitnahmeglied (38) des Führungsdrahts kraft- und/oder formschlüssig in Eingriff steht und in einer Einführstellung außer Eingriff steht.
13. Präparationsinstrument nach Satz 12, dadurch gekennzeichnet, dass das Mitnehmerelement (166) in Form eines Mitnehmervorsprungs (168) oder einer Mitnehmerausnehmung ausgebildet ist und dass das Führungsdrahtmitnahmeglied (38) korrespondierend ausgebildet ist in Form einer Führungsdrahtausnehmung (48) oder eines Führungsdrahtvorsprungs.
14. Präparationsinstrument nach Satz 13, dadurch gekennzeichnet, dass die Führungsdrahtausnehmung (48) in Form einer Nut (50), insbesondere in Form einer von der Instrumentenlängsachse (18) in radialer Richtung weg weisend geöffneten Ringnut (52), ausgebildet ist.
15. Präparationsinstrument nach einem der Sätze 12 bis 14, dadurch gekennzeichnet, dass das Mitnehmerelement (166) in radialer Richtung bezogen auf die Instrumentenlängsachse (18) bewegbar, insbesondere verschiebbar, angeordnet oder ausgebildet ist.
16. Präparationsinstrument nach einem der Sätze 12 bis 15, dadurch gekennzeichnet, dass das Mitnehmerelement (166) in der Mitnahmestellung mindestens teilweise in den Längskanal (24) hinein vorsteht.
17. Präparationsinstrument nach einem der Sätze 12 bis 16, dadurch gekennzeichnet, dass das Mitnehmerelement (166) von der Mitnahmestellung entgegen der Wirkung einer vom Präparationsinstrument (10) umfassten Rückstelleinrichtung (170) in die Einführstellung bewegbar ist.
18. Präparationsinstrument nach Satz 17, dadurch gekennzeichnet, dass die Rückstelleinrichtung (170) mindestens ein Rückstellelement (172) umfasst.
19. Präparationsinstrument nach Satz 18, dadurch gekennzeichnet, dass das mindestens eine Rückstellelement (172) in Form einer Feder (174), insbesondere in Form einer Schraubenfeder oder einer Blattfeder, ausgebildet ist.
20. Präparationsinstrument nach einem der Sätze 17 bis 19, dadurch gekennzeichnet, dass die Rückstelleinrichtung (170) in radialer Richtung auf die Instrumentenlängsachse (18) hin wirkend angeordnet oder ausgebildet ist.
21. Präparationsinstrument nach einem der Sätze 12 bis 20, dadurch gekennzeichnet, dass die Mitnahmeeinrichtung (132) in einer Grundstellung die Mitnahmestellung einnimmt.
22. Präparationsinstrument nach einem der Sätze 12 bis 21, dadurch gekennzeichnet, dass das Mitnehmerelement (166) in der Mitnahmestellung unter Vorspannung gehalten ist.
23. Präparationsinstrument nach einem der Sätze 12 bis 22, dadurch gekennzeichnet, dass die Mitnahmeeinrichtung (132) in Form einer Rast- oder Schnappverbindungseinrichtung (176) ausgebildet ist.
24. Präparationsinstrument nach einem der Sätze 12 bis 23, dadurch gekennzeichnet, dass die Mitnahmeeinrichtung (132) am proximalen Instrumententeil (30) angeordnet oder ausgebildet ist.
25. Präparationsinstrument nach einem der Sätze 12 bis 24, dadurch gekennzeichnet, dass die Mitnahmeeinrichtung (132) ein Betätigungsglied (178) umfasst, welches derart angeordnet oder ausgebildet ist, dass durch Einwirkung einer Betätigungskraft auf das Betätigungsglied (178) die Mitnahmeeinrichtung (132) von der Mitnahmestellung in die Einführstellung überführbar ist.
26. Präparationsinstrument nach Satz 25, dadurch gekennzeichnet, dass die Mitnahmeeinrichtung (132) ein Gehäuse (152) umfasst und dass das Betätigungsglied (178) zumindest in der Mitnahmestellung aus dem Gehäuse (152) vorsteht.
27. Präparationsinstrument nach Satz 25 oder 26, dadurch gekennzeichnet, dass das Betätigungsglied (178) und das Mitnehmerelement (166) einstückig, insbesondere monolithisch, ausgebildet sind.
28. Präparationsinstrument nach einem der voranstehenden Sätze, dadurch gekennzeichnet, dass das Präparationsinstrument (10) mindestens ein Werkzeugelement (62, 64, 66) zum Präparieren des Knochens (12) umfasst.
29. Präparationsinstrument nach Satz 28, dadurch gekennzeichnet, dass das mindestens eine Werkzeugelement (62, 64, 66) am distalen Instrumententeil (28) in distaler Richtung weisend angeordnet oder ausgebildet ist.
30. Präparationsinstrument nach Satz 28 oder 29, dadurch gekennzeichnet, dass das mindestens eine Werkzeugelement (62, 64, 66) in Form eines Perforationswerkzeugs (72) zum Perforieren der Kortikalis (74) des Knochens (12) ausgebildet ist.
31. Präparationsinstrument nach Satz 30, dadurch gekennzeichnet, dass das Perforationswerkzeug (72) mindestens eine Spitze (76), insbesondere eine Mehrzahl von Spitzen (76), umfasst und dass die mindestens eine Spitze (76) bezogen auf die Instrumentenlängsachse (18) in radialer Richtung versetzt angeordnet oder ausgebildet ist.
32. Präparationsinstrument nach Satz 31, dadurch gekennzeichnet, dass das Perforationswerkzeug (72) eine Mehrzahl von Spitzen (76) umfasst, welche die Instrumentenlängsachse (18) umgebend angeordnet oder ausgebildet sind.
33. Präparationsinstrument nach einem der Sätze 28 bis 32, dadurch gekennzeichnet, dass das mindestens eine Werkzeugelement (62, 64, 66) in Form eines Bohrwerkzeugs (78) zum Bohren eines Schraubenlochs (80) im Knochen (12) ausgebildet ist.
34. Präparationsinstrument nach Satz 33, dadurch gekennzeichnet, dass das Bohrwerkzeug (78) proximalseitig des Perforationswerkzeugs (72) angeordnet oder ausgebildet ist.
35. Präparationsinstrument nach einem der Sätze 28 bis 34, dadurch gekennzeichnet, dass das mindestens eine Werkzeugelement (62, 64, 66) in Form eines Gewindeschneidwerkzeugs (84) zum Schneiden eines Gewindes (86) in das Schraubenloch (80) im Knochen (12) ausgebildet ist.
36. Präparationsinstrument nach Satz 35, dadurch gekennzeichnet, dass das Gewindeschneidwerkzeug (84) proximalseitig des Bohrwerkzeugs (78) angeordnet oder ausgebildet ist.
37. Präparationsinstrument nach Satz 35 oder 36, dadurch gekennzeichnet, dass das Bohrwerkzeug (78) eine Bohrwerkzeuglänge aufweist, dass das Gewindeschneidwerkzeug (84) eine Gewindeschneidwerkzeuglänge aufweist und dass ein Verhältnis von Gewindeschneidwerkzeuglänge und Bohrwerkzeuglänge höchstens etwa 1 beträgt.
38. Präparationsinstrument nach einem der Sätze 28 bis 37, dadurch gekennzeichnet, dass der distale Instrumententeil (28) einen distalen Schaft (56) umfasst und dass sich das mindestens eine Werkzeugelement (62, 64, 66) in distaler Richtung weisend vom distalen Schaft (56) weg erstreckt.
39. Präparationsinstrument nach Satz 38, dadurch gekennzeichnet, dass ein Außendurchmesser (68) des distalen Schafts (56) größer ist als ein vom mindestens einen Werkzeugelement (62, 64, 66) definierter Werkzeugaußendurchmesser (70).
40. Präparationsinstrument nach Satz 38 oder 39, dadurch gekennzeichnet, dass der Innengewindeabschnitt (116) im Bereich des distalen Schafts (56) angeordnet oder ausgebildet ist.
41. Präparationsinstrument nach einem der voranstehenden Sätze, dadurch gekennzeichnet, dass das Präparationsinstrument (10) eine Einführtiefenmesseinrichtung (122) umfasst zum Bestimmen eines Abstands (124) des distalen Endes (24) des aus dem distalen Ende (20) des Präparationsinstruments (10) vorstehenden Führungsdrahts (22) vom distalen Ende (20) des Präparationsinstruments (10).
42. Präparationsinstrument nach Satz 41, dadurch gekennzeichnet, dass die Einführtiefenmesseinrichtung (122) eine Mehrzahl von Markierungselementen (126) umfasst und dass die Markierungselemente (126) proximalseitig des vom proximalen Instrumententeil (30) umfassten Gewindeabschnitts (114) am proximalen Instrumententeil (30) angeordnet oder ausgebildet sind.
43. Präparationsinstrument nach einem der Sätze 4 bis 42, dadurch gekennzeichnet, dass das Präparationsinstrument (10) einen distalen Instrumentengriff (96) umfasst zum Drehen des distalen Instrumententeils (28) um die Instrumentenlängsachse (18).
44. Präparationsinstrument nach einem der Sätze 43, dadurch gekennzeichnet, dass der distale Instrumentengriff (96) zwei Griffarme (188, 190) umfasst, welche in radialer Richtung und diametral voneinander weg weisen.
45. Präparationsinstrument nach Satz 44, dadurch gekennzeichnet, dass der distale Instrumentengriff (96) mit dem distalen Instrumententeil (28) lösbar verbindbar ausgebildet ist.
46. Präparationsinstrument nach Satz 44 oder 45, dadurch gekennzeichnet, dass der distale Instrumentengriff (96) mit dem distalen Instrumententeil (28) in einer Verbindungsstellung bezogen auf die Instrumentenlängsachse (18) drehfest gekoppelt ist.
47. Präparationsinstrument nach einem der Sätze 44 bis 46, dadurch gekennzeichnet, dass das Präparationsinstrument (10) eine Verbindungseinrichtung (94) zum kraft- und/oder formschlüssigen Koppeln des distalen Instrumentengriffs (96) und des distalen Instrumententeils (28) in der Verbindungsstellung umfasst.
48. Präparationsinstrument nach Satz 47, dadurch gekennzeichnet, dass die Verbindungseinrichtung (94) einen distalen Anschlag (102) umfasst zum Begrenzen einer Bewegung des distalen Instrumentengriffs (96) relativ zum distalen Instrumententeil (28) in der Verbindungsstellung in distaler Richtung.
49. Präparationsinstrument nach Satz 48, dadurch gekennzeichnet, dass der distale Anschlag (94) eine sich quer, insbesondere senkrecht, zur Instrumentenlängsachse (18) erstreckende, in proximaler Richtung weisende distale Anschlagfläche (104) umfasst, an welcher der distale Instrumentengriff (96) in der Verbindungsstellung anliegt.
50. Präparationsinstrument nach einem der Sätze 47 bis 49, dadurch gekennzeichnet, dass die Verbindungseinrichtung (94) erste und zweite, zusammenwirkende Verbindungselemente (90, 92) umfasst, welche einerseits am distalen Instrumententeil (28) und andererseits am distalen Instrumentengriff (96) angeordnet oder ausgebildet sind, und dass die ersten und zweiten Verbindungselemente (90, 92) in der Verbindungsstellung kraft- und/oder formschlüssig in Eingriff stehen und in einer Reinigungsstellung außer Eingriff stehen.
51. Präparationsinstrument nach Satz 50, dadurch gekennzeichnet, dass eines der zusammenwirkenden Verbindungselemente (90, 92) in Form einer Verzahnung (106) ausgebildet ist, insbesondere mit einer Mehrzahl von Zähnen (108), und dass eines der Verbindungselemente (90 ,92) in Form mindestens eines in der Verbindungsstellung in die Verzahnung (106) eingreifenden Verbindungsvorsprungs (110) ausgebildet ist.
52. Präparationsinstrument nach Satz 50 oder 51, dadurch gekennzeichnet, dass die ersten und zweiten Verbindungselemente (90, 92) in einer Drehstellung relativ zueinander um die Instrumentenlängsachse (18) verdrehbar sind.
53. Präparationsinstrument nach Satz 52, dadurch gekennzeichnet, dass der mindestens eine Verbindungsvorsprung (110) in der Drehstellung in radialer Richtung bezogen auf die Instrumentenlängsachse (18) auslenkbar angeordnet oder ausgebildet ist.
54. Präparationsinstrument nach einem der Sätze 51 bis 53, dadurch gekennzeichnet, dass der mindestens eine Verbindungsvorsprung (110) an einem freien Ende eines Federelements (204), insbesondere eines Blattfederelements, angeordnet oder ausgebildet ist.
55. Präparationsinstrument nach einem der Sätze 51 bis 54, dadurch gekennzeichnet, dass die Verbindungseinrichtung (94) einen Verbindungsring (192) umfasst, welcher die in radialer Richtung weisende Verzahnung (106) umgibt und aufweitbar ausgebildet ist.
56. Präparationsinstrument nach Satz 55, dadurch gekennzeichnet, dass der Verbindungsring (192) einen sich in Umfangsrichtung bezogen auf die Instrumentenlängsachse (18) erstreckenden Aufweitspalt (194) umfasst, dass der eine Griffarm (190) des distalen Instrumentengriffs (96) über zwei mit jeweils einem von zwei freien, aufeinander zu weisenden Enden des Verbindungsrings (192) verbundenen Griffarmstegen (200, 202) verbunden ist.
57. Präparationsinstrument nach Satz 56, dadurch gekennzeichnet, dass die zwei Griffarmstege (200, 202) parallel zueinander verlaufen.
58. Präparationsinstrument nach Satz 56 oder 57, dadurch gekennzeichnet, dass das Federelement (204) zwischen den beiden Griffarmstegen (200, 202) angeordnet oder ausgebildet ist.
59. Präparationsinstrument nach einem der Sätze 56 bis 58, dadurch gekennzeichnet, dass der eine Griffarm (188) am Verbindungsring (192) dem Aufweitspalt (194) bezogen auf die Instrumentenlängsachse (18) diametral gegenüberliegend angeordnet ist.
60. Präparationsinstrument nach einem der Sätze 47 bis 59, dadurch gekennzeichnet, dass die Verbindungseinrichtung (94) eine Sicherungseinrichtung (210) umfasst Sichern der ineinandergreifenden ersten und zweiten Verbindungselemente (90, 92) in der Verbindungsstellung.
61. Präparationsinstrument nach Satz 60, dadurch gekennzeichnet, dass die Sicherungseinrichtung (210) ein Sicherungselement (212) umfasst, welches am distalen Instrumentengriff (96) oder am distalen Instrumententeil (28) von einer Entsicherungsstellung, in welcher der distale Instrumentengriff (96) und der distale Instrumententeil (30) relativ zueinander in einer Einschraubdrehrichtung bezogen auf die Instrumentenlängsachse (18) verdrehbar sind, in eine Sicherungsstellung bewegbar ist, in welcher der distale Instrumentengriff (96) und der distale Instrumententeil (28) relativ zueinander in der Einschraubdrehrichtung bezogen auf die Instrumentenlängsachse (18) unverdrehbar sind.
62. Präparationsinstrument nach Satz 61, dadurch gekennzeichnet, dass das Sicherungselement (212) in Form einer Sicherungshülse (214) ausgebildet ist, welche auf einem Griffarm (190) des distalen Instrumentengriffs (96) in radialer Richtung bezogen auf die Instrumentenlängsachse (18), insbesondere auf diese hin und von dieser weg, bewegbar, insbesondere verschiebbar, angeordnet oder ausgebildet ist.
63. Präparationsinstrument nach Satz 61 oder 62, dadurch gekennzeichnet, dass das Sicherungselement (212) ausgebildet ist zum Verhindern eines Aufweitens des Verbindungsrings (192) in der Sicherungsstellung.
64. Präparationsinstrument nach einem der Sätze 61 bis 63, dadurch gekennzeichnet, dass das Sicherungselement (212) die Griffarmstege (200, 202) umschließt und in der Sicherungsstellung eine der Instrumentenlängsachse (18) maximal angenäherte Position und in der Entsicherungsstellung eine von der Instrumentenlängsachse (18) maximal entfernte Position einnimmt.
65. Präparationsinstrument nach einem der Sätze 4 bis 64, dadurch gekennzeichnet, dass das Präparationsinstrument (10) einen proximalen Instrumentengriff (134) umfasst zum Drehen des proximalen Instrumententeils (30) um die Instrumentenlängsachse (18).
66. Präparationsinstrument nach Satz 65, dadurch gekennzeichnet, dass der proximale Instrumentengriff (134) mit dem proximalen Instrumententeil (30) in einer Kopplungsstellung bezogen auf die Instrumentenlängsachse (18) drehfest gekoppelt ist.
67. Präparationsinstrument nach Satz 65 oder 66, dadurch gekennzeichnet, dass das Präparationsinstrument (10) eine Kopplungseinrichtung (136) umfasst zum kraft- und/oder formschlüssigen Koppeln des proximalen Instrumentengriffs (134) und des proximalen Instrumententeils (30) in der Kopplungsstellung.
68. Präparationsinstrument nach Satz 67, dadurch gekennzeichnet, dass die Kopplungseinrichtung (136) erste und zweite, zusammenwirkende Kopplungselemente (138, 140) umfasst, welche einerseits am proximalen Instrumententeil (30) und andererseits am proximalen Instrumentengriff (134) angeordnet oder ausgebildet sind, und dass die ersten und zweiten Kopplungselemente (138, 140) in der Kopplungsstellung kraft- und/oder formschlüssig in Eingriff stehen und in einer Entkopplungsstellung außer Eingriff stehen.
69. Präparationsinstrument nach Satz 68, dadurch gekennzeichnet, dass die Kopplungseinrichtung (136) einen proximalen Anschlag (148) umfasst zum Begrenzen einer Bewegung des proximalen Instrumentengriffs (134) relativ zum proximalen Instrumententeil (30) in der Kopplungsstellung in distaler Richtung.
70. Präparationsinstrument nach Satz 69, dadurch gekennzeichnet, dass der proximale Anschlag (148) eine sich quer, insbesondere senkrecht, zur Instrumentenlängsachse (18) erstreckende, in proximaler Richtung weisende proximale Anschlagfläche (150) umfasst, an welcher der proximale Instrumentengriff (134) in der Kopplungsstellung anliegt.
71. Präparationsinstrument nach Satz 70, dadurch gekennzeichnet, dass die Mitnahmeeinrichtung (132) die proximale Anschlagfläche (150) definiert oder umfasst.
72. Präparationsinstrument nach einem der Sätze 68 bis 71, dadurch gekennzeichnet, dass das eine Kopplungselement (138) in Form eines Kopplungsvorsprungs (142) und das andere Kopplungselement (138) in Form einer zum Kopplungsvorsprung (142) korrespondierenden Kopplungsausnehmung (144) ausgebildet ist.
73. Präparationsinstrument nach Satz 72, dadurch gekennzeichnet, dass der Kopplungsvorsprung (142) in Form eines Außenvielrund oder Außenmehrkant (146) ausgebildet ist.
74. Präparationsinstrument nach einem der Sätze 68 bis 73, dadurch gekennzeichnet, dass eines der zusammenwirkenden Kopplungselemente (138, 140) im Bereich eines proximalen Endes des proximalen Instrumententeils (30) angeordnet oder ausgebildet ist.
75. Präparationsinstrument nach einem der Sätze 4 bis 74, dadurch gekennzeichnet, dass ein Innendurchmesser des Längskanals (24) im Bereich des proximalen Instrumententeils (30) einem Außendurchmesser des Führungsdrahts (22) entspricht.
76. Präparationsinstrument nach einem der Sätze 28 bis 75, dadurch gekennzeichnet, dass ein Innendurchmesser des Längskanals (24) im Bereich des mindestens einen Werkzeugelements (62, 64, 66) einem Außendurchmesser des Führungsdrahts (22) entspricht.
77. Verfahren zum Präparieren eines künstlichen Knochenmodells oder eines menschlichen oder tierischen Knochens vor der Verankerung einer Knochenschraube (14), insbesondere einer Pedikelschraube, bei welchem Verfahren ein Schraubenloch (80) im Knochenmodell oder im Knochen präpariert wird, dadurch gekennzeichnet, dass zum Präparieren ein Präparationsinstrument (10) nach einem der voranstehenden Sätze verwendet wird.

Die nachfolgende Beschreibung einer bevorzugten Ausführungsform der Erfindung dient im Zusammenhang mit den Zeichnungen der näheren Erläuterung. Es zeigen:
- Figur 1:: eine schematische perspektivische Ansicht eines Teils eines Ausführungsbeispiels eines Präparationsinstrument;
- Figur 2:: eine teilweise Schnittansicht der Anordnung aus Figur 1;
- Figur 3:: eine vergrößerte Teilansicht des Bereichs A aus Figur 2;
- Figur 4:: eine Ansicht des Präparationsinstruments aus Figur 1 mit gekoppelten Griffen und eingeführtem Führungsdraht;
- Figur 5:: eine vergrößerte Teilansicht des Bereichs B aus Figur 4;
- Figur 6:: eine schematische Ansicht der Anordnung aus Figur 4 beim Perforieren der Kortikalis eines Knochens;
- Figur 7:: eine schematische perspektivische Ansicht der Anordnung aus Figur 4 beim Eintreiben des Führungsdrahts in den Knochen;
- Figur 8:: eine vergrößerte Teilansicht des Bereichs C aus Figur 7;
- Figur 9:: eine schematische Ansicht ähnlich Figur 7 beim Bohren eines Schraubenlochs in den Knochen;
- Figur 10:: eine vergrößerte Teilansicht des Bereichs D aus Figur 9;
- Figur 11:: eine schematische Ansicht der Anordnung ähnlich Figur 9 beim Zurückziehen des Präparationsinstruments vom Führungsdraht nach dem Entkoppeln von diesem;
- Figur 12:: eine schematische Darstellung des Einführens einer Knochenschraube mittels des Führungsdrahts in das präparierte Schraubenloch sowie Einschrauben der Knochenschraube in dieses;
- Figur 13:: eine Seitenansicht des distalen Instrumententeils;
- Figur 14:: eine Schnittansicht längs Linie 14-14 in Figur 13;
- Figur 15:: eine perspektivische vergrößerte Teilansicht des Bereichs E aus Figur 13;
- Figur 16:: eine perspektivische Ansicht des distalen Instrumententeils mit daran gekoppeltem distalen Instrumentengriff;
- Figur 17:: eine Draufsicht auf die Anordnung aus Figur 16 in der Sicherungsstellung;
- Figur 18:: eine Draufsicht auf die Anordnung aus Figur 16 in der Entsicherungsstellung;
- Figur 19:: eine perspektivische Ansicht des proximalen Instrumententeils; und
- Figur 20:: eine Explosionsdarstellung der Anordnung aus Figur 19 mit Führungsdraht.

In den Figuren ist schematisch ein insgesamt mit dem Bezugszeichen 10 bezeichnetes Ausführungsbeispiel eines Präparationsinstruments dargestellt. Es ist wie nachfolgend noch näher erläutert werden wird zum Präparieren eines Knochens 12 vor dem Verankern einer Knochenschraube 14 im Knochen 12 ausgebildet.

Das Präparationsinstrument 10 umfasst einen Instrumentenschaft 16, welcher eine Instrumentenlängsachse 18 definiert. Das Präparationsinstrument 10 weist ein distales Ende 20 auf und umfasst einen vom Instrumentenschaft 16 separat ausgebildeten und vollständig trennbaren Führungsdraht 22. Dieser kann insbesondere in Form eines sogenannten K-Drahts ausgebildet sein.

Das Präparationsinstrument 10 ist ausgebildet, um verschiedene Präparationsschritte durchzuführen. Insbesondere ist das Präparationsinstrument 10 zum Verankern des Führungsdrahts 22 im Knochen 12 ausgebildet.

Das Präparationsinstrument 10 umfasst einen sich parallel zur Instrumentenlängsachse 18 erstreckenden Längskanal 24. Der Längskanal 24 durchsetzt das Präparationsinstrument 10 durchgängig vom distalen Ende 20 bis zu einem proximalen Ende 26 desselben. Der Längskanal 24 ist ausgebildet und derart bemessen, dass der Führungsdraht 22 in den Längskanal 24 eingeführt und durch diesen hindurchgeschoben werden kann.

Das Präparationsinstrument 10 umfasst einen distalen Instrumententeil 28 und einen proximalen Instrumententeil 30. Die beiden Instrumententeile 28 und 30 sind miteinander koppelbar und relativ zueinander bewegbar ausgebildet. Hierfür dient insbesondere eine Bewegungsgewindeverbindung 32. Der distale und der proximale Instrumententeil 28, 30 sind über die Bewegungsgewindeverbindung 32 miteinander verschraubbar koppelbar. Der genaue Aufbau und die Funktion der Bewegungsgewindeverbindung 32 werden nachfolgend noch im Einzelnen erläutert.

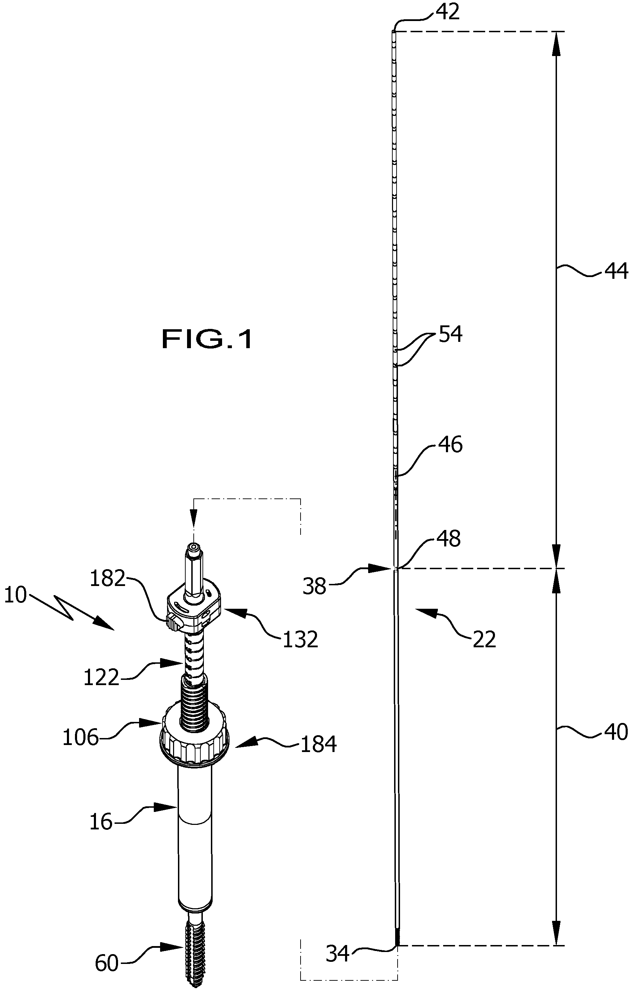

Der Führungsdraht 22 ist wie schematisch in Figur 1 dargestellt stabförmig ausgebildet. Ausgehend von einem distalen Ende 34 ist ein kurzer Außengewindeabschnitt 36 ausgebildet.

Ferner ist am Führungsdraht 22 ein Führungsdrahtmitnahmeglied 38 ausgebildet. Dieses weist vom distalen Ende 34 einen Abstand 40 auf. Ferner weist es von einem proximalen Ende 42 des Führungsdrahts 22 einen Abstand 44 auf. Der Führungsdraht 22 definiert ferner eine Führungsdrahtlängsachse 46.

Das Führungsdrahtmitnahmeglied 38 ist in Form einer Führungsdrahtausnehmung 48 ausgebildet. Es ist in Form einer Nut 50 ausgebildet, und zwar in Form einer von der Instrumentenlängsachse 18 in radialer Richtung weg weisend geöffneten Ringnut 52.

Ein Verhältnis des Abstands 40 zum Abstand 44 beträgt beim dargestellten Ausführungsbeispiel etwa 2:3. Am Führungsdraht 22 sind im Bereich zwischen dem Führungsdrahtmitnahmeglied 38 und dem proximalen Ende 42 Markierungen 54 in Form von Ringen angebracht, beispielsweise durch Laserbeschriften des aus einem Instrumentenstahl oder einem anderen biokompatiblen metallischen Werkstoff ausgebildeten Führungsdrahts 22.

Der distale Instrumententeil 28 umfasst einen distalen Schaft 56. Dieser ist hülsenförmig ausgebildet.

Von einem distalen Ende 58 des distalen Schafts 56 erstreckt sich in distaler Richtung weg ein Werkzeugkörper 60, welcher mehrere Werkzeugelemente 62, 64 und 66 umfasst. Der Werkzeugkörper 60 ist ähnlich einer Knochenschraube geformt.

Ein Außendurchmesser 68 des distalen Schafts 56 ist größer als ein vom Werkzeugkörper 60 und damit von den Werkzeugelementen 62, 64, 66 definierter Werkzeugaußendurchmesser 70.

Die vom Präparationsinstrument 10 umfassten Werkzeugelemente 62, 64 und 66 sind zum Präparieren des Knochens 12 ausgebildet. Die drei Werkzeugelemente 62, 64, 66 sind am Werkzeugkörper 60 und somit am distalen Instrumententeil 28 in distaler Richtung weisend angeordnet beziehungsweise ausgebildet.

Das Werkzeugelement 62 ist in Form eines Perforationswerkzeugs 72 zum Perforieren der Kortikalis 74 des Knochens 12 ausgebildet. Hierfür weist das Perforationswerkzeug 72 mehrere Spitzen 76 auf. Diese sind bezogen auf die Instrumentenlängsachse 18 in radialer Richtung versetzt angeordnet beziehungsweise ausgebildet. Bei dem in den Figuren schematisch dargestellten Ausführungsbeispiel des Präparationsinstruments 10 sind insgesamt fünf Spitzen 76 vorgesehen, welche die Instrumentenlängsachse 18 umgebend angeordnet beziehungsweise ausgebildet sind. Die Spitzen 76 bilden gemeinsam das Perforationswerkzeug 72, welches geeignet ist, die harte Kortikalis 74 zu durchschneiden und einen kleinen Kortikalispfropf auszustanzen.

Das Werkzeugelement 64 ist in Form eines Bohrwerkzeugs 78 zum Bohren eines Schraubenlochs 80 im Knochen 12 ausgebildet. Hierfür sind am Werkzeugkörper 60 Bohrschneiden 82 ausgebildet, die geeignet sind, um bei einer Verdrehung des Werkzeugkörpers 60 in den Knochen 12 hinein das Schraubenloch 80 zu präparieren.

Das Bohrwerkzeug 78 ist proximalseitig des Perforationswerkzeugs 72 angeordnet beziehungsweise ausgebildet. Bei dem in den Figuren dargestellten Ausführungsbeispiel schließt sich das Bohrwerkzeug 78 unmittelbar an das Perforationswerkzeug 72 an.

Das Werkzeugelement 66 ist in Form eines Gewindeschneidwerkzeugs 84 zum Schneiden eines Gewindes 86 in das Schraubenloch 80 im Knochen 12 ausgebildet. Das Gewindeschneidwerkzeug 84 umfasst ein Schneidgewinde 88. Bei dem in den Figuren dargestellten Ausführungsbeispiel ist das Gewindeschneidwerkzeug 84 proximalseitig des Bohrwerkzeugs 78 angeordnet beziehungsweise ausgebildet.

Bei einem Ausführungsbeispiel weist das Bohrwerkzeug 78 eine Bohrwerkzeuglänge auf. Das Gewindeschneidwerkzeug 84 weist eine Gewindeschneidwerkzeuglänge auf. Ein Verhältnis von Gewindeschneidwerkzeuglänge und Bohrwerkzeuglänge beträgt bei diesem Ausführungsbeispiel höchstens etwa 1. Mithin ist also die Gewindeschneidwerkzeuglänge nicht größer als die Bohrwerkzeuglänge.

Ein proximales Ende des distalen Instrumententeils 28 wird durch ein erstes Verbindungselement 90 gebildet, welches zusammenwirkend mit einem zweiten Verbindungselement 92 eine Verbindungseinrichtung 94 zum kraft- und/oder formschlüssigen Koppeln des distalen Instrumententeils 28 mit einem distalen Instrumentengriff in einer Verbindungsstellung bildet.

Das erste Verbindungselement 90 umfasst einen zylindrischen Körper 98, welcher einen größeren Außendurchmesser aufweist als der distale Schaft 56. An den Körper 98 schließt sich distalseitig ein in radialer Richtung vorstehender Ringflansch 100 an. Der Ringflansch 100 bildet einen distalen Anschlag 102 zum Begrenzen einer Bewegung des distalen Instrumentengriffs 96 relativ zum distalen Instrumententeil 28 in der Verbindungsstellung in distaler Richtung.

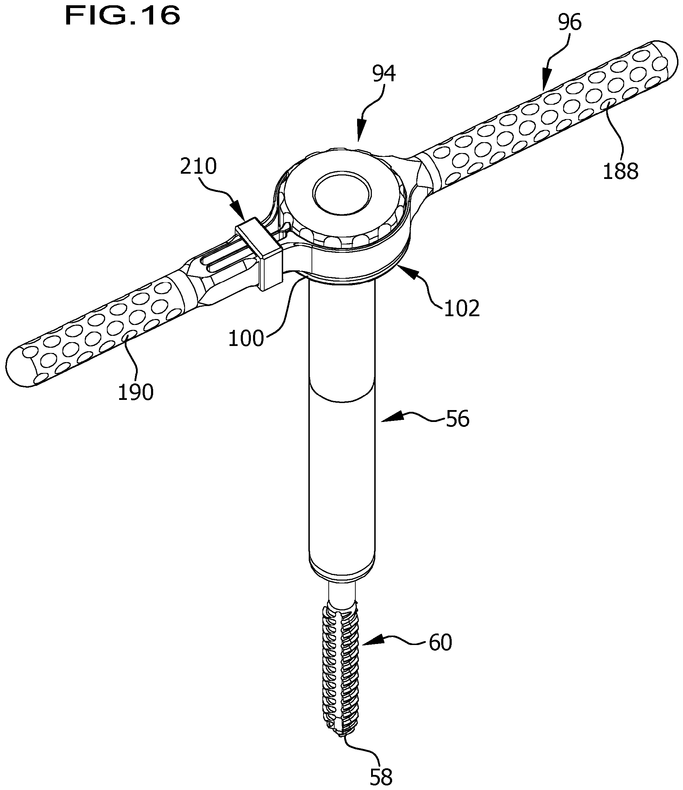

Der distale Anschlag 102 umfasst eine distale Anschlagfläche 104, welche sich quer, beim in den Figuren dargestellten Ausführungsbeispiel senkrecht, zur Instrumentenlängsachse 18 erstreckt und in proximaler Richtung weist. Der distale Instrumentengriff 96 liegt in der Verbindungsstellung, wie beispielsweise in Figur 16 schematisch dargestellt, an der Anschlagfläche 104 an.

Der Körper 98 des ersten Verbindungselements 90 ist in radialer Richtung weg weisend mit einer umlaufenden Verzahnung 106 ausgebildet, welche eine Mehrzahl von Zähnen 108 umfasst. Die Zähne 108 sind korrespondierend zum zweiten Verbindungselement ausgebildet, welches als ein in die Verzahnung 106 eingreifender Verbindungsvorsprung 110 ausgebildet ist. Dessen genaue Anordnung am distalen Instrumentengriff 96 sowie sein Zusammenwirken mit dem ersten Verbindungselement 90 werden nachfolgend noch im Einzelnen erläutert werden.

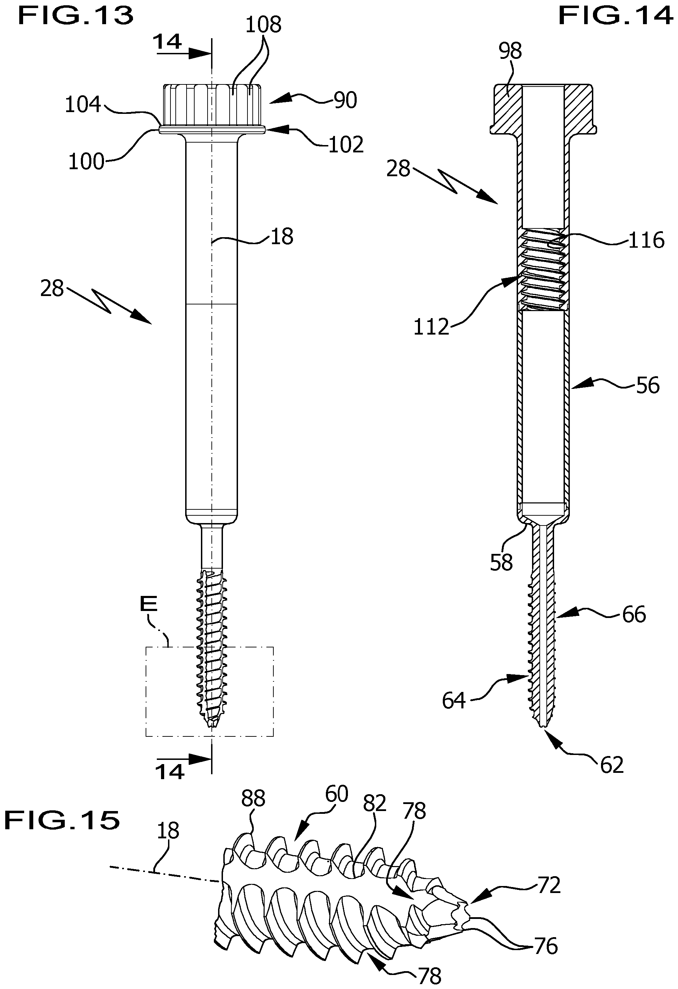

Der distale Instrumententeil 28 umfasst noch eine weitere Komponente, nämlich einen von zwei zusammenwirkenden Gewindeabschnitten 112 und 114 der Bewegungsgewindeverbindung 32. Am distalen Instrumententeil 28 ist der Gewindeabschnitt 112 angeordnet beziehungsweise ausgebildet, und zwar im Inneren des distalen Schafts 56 als. Der Innengewindeabschnitt 116 korrespondiert zu einem Außengewindeabschnitt 118, welcher den anderen Gewindeabschnitt 114 bildet. Wie insbesondere in Figur 14 gut zu erkennen, ist der Innengewindeabschnitt 116 beabstandet zum ersten Verbindungselement 90 einerseits und beabstandet zum distalen Ende 58 andererseits ausgebildet. Eine Länge des Innengewindeabschnitts 116 parallel zur Instrumentenlängsachse 18 beträgt etwa 1/5 einer Gesamtlänge des distalen Schafts 56 einschließlich des ersten Verbindungselements 90.

Der proximale Instrumententeil 30 ist schaftförmig ausgebildet und weist ausgehend von einem distalen Ende 120 den Außengewindeabschnitt 118 auf. Der Außengewindeabschnitt 118 erstreckt sich auf nahezu einer halben Länge des proximalen Instrumententeils 30 in proximaler Richtung.

An den Außengewindeabschnitt 118 schließt sich proximalseitig eine vom Präparationsinstrument 10 umfasste Einführtiefenmesseinrichtung 122 an. Diese ist ausgebildet zum Bestimmen eines Abstands 124 des distalen Endes 34 des Führungsdrahts 22 vom distalen Ende 20 des Präparationsinstruments 10. Die Einführtiefenmesseinrichtung 122 umfasst eine Mehrzahl von Markierungselementen 126, welche proximalseitig des vom proximalen Instrumententeil 30 umfassten Gewindeabschnitts 114 am proximalen Instrumententeil 30 angeordnet oder ausgebildet sind. Die Markierungselemente 126 sind in Form von äquidistant in Richtung der Instrumentenlängsachse 18 beabstandeten Ringen 128 ausgebildet und zusätzlich mit einer Beschriftung 130 versehen, welche zu jedem der Ringe 128 den Abstandswert des Abstands 124 angibt.

Wie erläutert umfasst beim in den Figuren dargestellten Ausführungsbeispiel der distale Instrumententeil 28 den Innengewindeabschnitt 116. Der proximale Instrumententeil 30 umfasst den Außengewindeabschnitt 118.

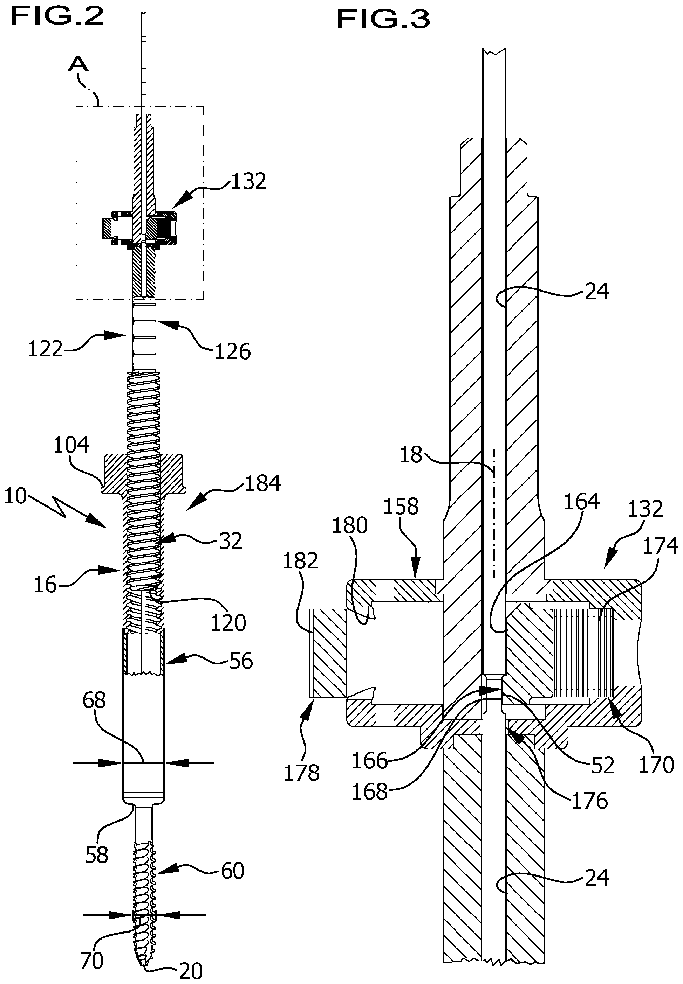

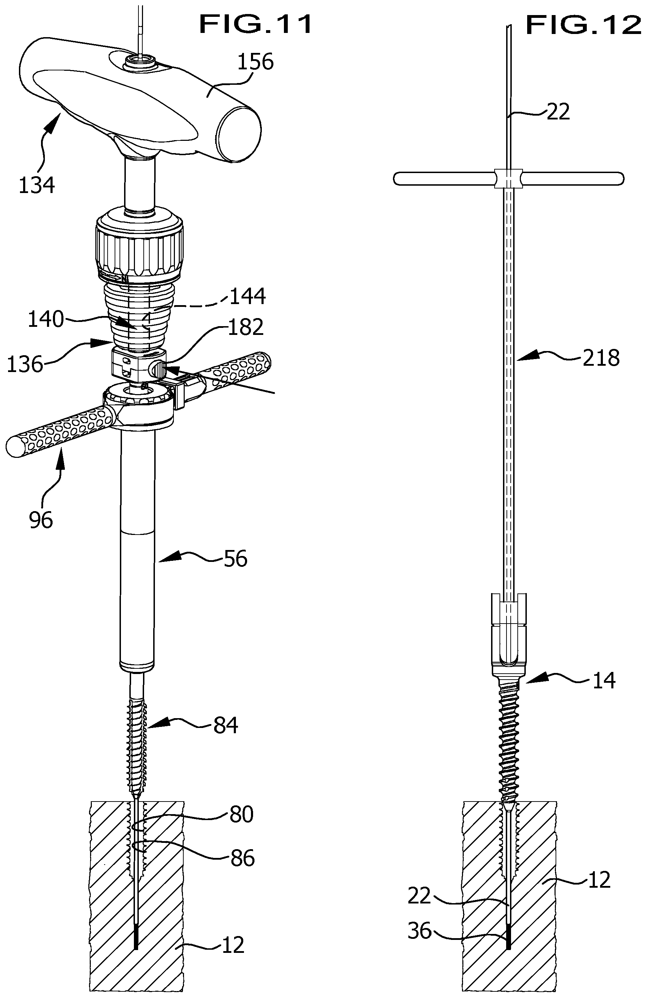

Proximalseitig schließt sich an die Einführtiefenmesseinrichtung 122 eine Mitnahmeeinrichtung 132 an. Sie ist ausgebildet, um mit dem Führungsdraht 22 in einer Mitnahmestellung kraft- und/oder formschlüssig bezogen auf die Instrumentenlängsachse 18 in axialer Richtung gekoppelt zu werden. In der Mitnahmestellung wie schematisch in den Figuren 2 und 3 dargestellt stehen die Mitnahmeeinrichtung 132 und der Führungsdraht 22 in axialer Richtung gekoppelt in Eingriff. In einer Einführstellung wie sie schematisch in Figur 11 dargestellt ist stehen die Mitnahmeeinrichtung 132 und der Führungsdraht 22 außer Eingriff, sodass das Präparationsinstrument 10 und der Führungsdraht 22 relativ zueinander in axialer Richtung, also parallel zur Instrumentenlängsachse 18, relativ zueinander bewegbar sind. Der Aufbau der Mitnahmeeinrichtung 132 wird im Einzelnen nachfolgend noch näher erläutert werden.

Das Präparationsinstrument 10 umfasst ferner einen proximalen Instrumentengriff 134 zum Drehen des proximalen Instrumententeils 30 um die Instrumentenlängsachse 18.

Der proximale Instrumentengriff 134 ist mit dem proximalen Instrumententeil 30 in einer schematisch in Figur 11 dargestellten Kopplungsstellung bezogen auf die Instrumentenlängsachse 18 zumindest in einer Drehrichtung drehfest gekoppelt.

Das Präparationsinstrument 10 umfasst eine Kopplungseinrichtung 136 zum kraft- und/oder formschlüssigen Koppeln des proximalen Instrumentengriffs 134 und des proximalen Instrumententeils 30 in der Kopplungsstellung.

Die Kopplungseinrichtung 136 umfasst erste und zweite, zusammenwirkende Kopplungselemente 138 und 140. Das erste Kopplungselement 138 ist am proximalen Instrumententeil 30 angeordnet beziehungsweise ausgebildet. Das zweite Kopplungselement 140 ist am proximalen Instrumentengriff 134 angeordnet beziehungsweise ausgebildet. Die ersten und zweiten Kopplungselemente 138, 140 stehen in der Kopplungsstellung kraft- und/oder formschlüssig in Eingriff und in der Entkopplungsstellung außer Eingriff.

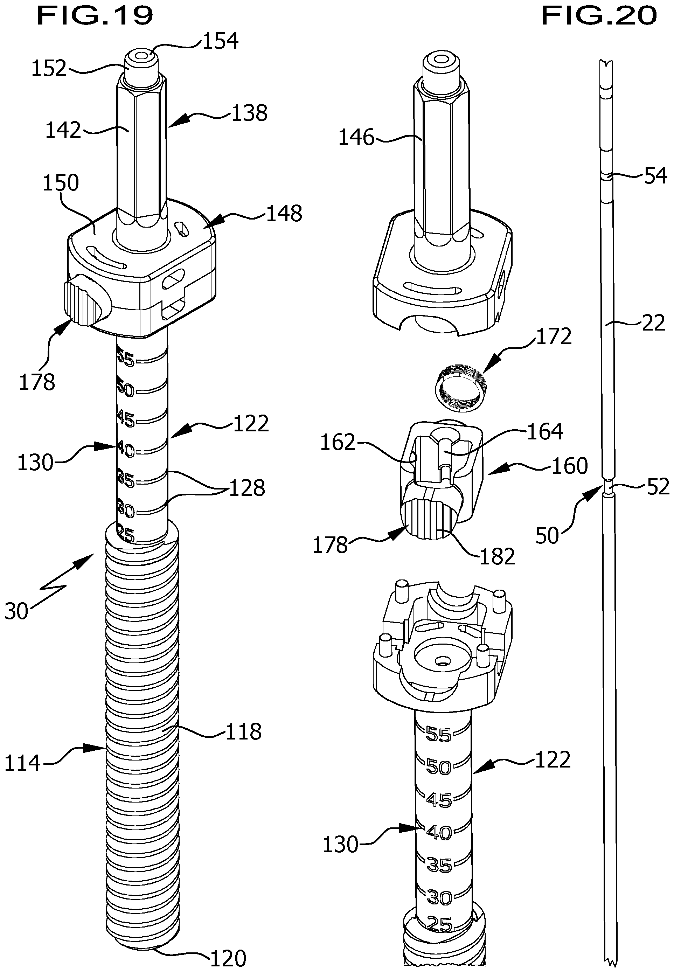

Das erste Kopplungselement 138 ist in Form eines Kopplungsvorsprungs 142 ausgebildet. Das zweite Kopplungselement 140 ist in Form einer zum Kopplungsvorsprung 142 korrespondierenden Kopplungsausnehmung 144 ausgebildet. Wie schematisch in Figur 19 dargestellt ist der Kopplungsvorsprung 142 in Form eines Außenmehrkant 146 ausgebildet. Die Kopplungsausnehmung 144 ist entsprechend in Form eines Innenmehrkant ausgebildet. Bei einem alternativen Ausführungsbeispiel sind der Kopplungsvorsprung 142 in Form eines Au-ßenvielrund und die Kopplungsausnehmung 144 in Form eines Innenvielrund ausgebildet.

Der Kopplungsvorsprung 142, mithin also das erste Kopplungselement 138, schließen sich proximalseitig an die Mitnahmeeinrichtung 132 an. Somit ist das erste Kopplungselement 138 im Bereich eines proximalen Endes des proximalen Instrumententeils 30 angeordnet beziehungsweise ausgebildet.

Die Kopplungseinrichtung 136 umfasst ferner einen proximalen Anschlag 148 zum Begrenzen einer Bewegung des proximalen Instrumentengriffs 134 relativ zum proximalen Instrumententeil 30 in der Kopplungsstellung in distaler Richtung.

Der proximale Anschlag 148 umfasst eine sich quer, bei dem in den Figuren dargestellten Ausführungsbeispiel senkrecht, zur Instrumentenlängsachse 18 erstreckende, in proximaler Richtung weisende proximale Anschlagfläche 150, an welcher der proximale Instrumentengriff 134 in der Kopplungsstellung anliegt wie schematisch in Figur 11 dargestellt. Bei dem in den Figuren dargestellten Ausführungsbeispiel definiert die Mitnahmeeinrichtung 132 die proximale Anschlagfläche 150 beziehungsweise umfasst diese.

Proximalseitig des Außenmehrkant 146 ist eine kurze zylindrische Hülse 152 ausgebildet, welche ein proximales Ende 154 des proximalen Instrumententeils 30 definiert.

Der proximale Instrumentengriff 134 ist in Form eines Ratschengriffs ausgebildet, welcher wahlweise im Uhrzeigersinn oder im Gegenuhrzeigersinn drehfest mit dem proximalen Instrumententeil 30 koppelbar ist, um diesen in entsprechender Drehrichtung um die Instrumentenlängsachse 18 zu verdrehen. Bei der Ausgestaltung als Ratschengriff ist dann eine Verdrehung des proximalen Instrumentengriffs 134 relativ zum proximalen Instrumententeil 30 auch in der Kopplungsstellung möglich, also wenn der proximale Instrumentengriff 134 kraft- und/oder formschlüssig mit dem proximalen Instrumententeil 30 gekoppelt ist.

Der proximale Instrumentengriff 134 umfasst ein sich quer zur Instrumentenlängsachse 18 erstreckendes Griffelement 156, sodass der proximale Instrumentengriff 134 eine im Wesentlichen T-förmige Gestalt aufweist.

Der Aufbau der Mitnahmeeinrichtung 132 wird nachfolgend in Verbindung mit den Figuren 2, 3 sowie 19 und 20 näher erläutert.

Die Mitnahmeeinrichtung 132 umfasst ein Gehäuse 158, welches im Wesentlichen quaderförmig ausgebildet ist. Im Gehäuse 158 ist ein Mitnehmerglied 160 in einer Richtung quer, nämlich senkrecht, zur Instrumentenlängsachse 18 verschiebbar angeordnet. Das Mitnehmerglied 160 ist im Wesentlichen ringförmig ausgebildet und umfasst eine sich parallel zur Instrumentenlängsachse 18 erstreckende Durchgangsöffnung 162. Die Durchgangsöffnung 162 umfasst einen Bereich, welcher einen Längskanalabschnitt 164 definiert. Der Längskanalabschnitt 164 ist einseitig offen ausgestaltet, nämlich in Richtung auf die Instrumentenlängsachse 18 hin weisend.

Vom Längskanalabschnitt 164 steht ein Mitnehmerelement 166 etwas in Richtung auf die Instrumentenlängsachse 18 hin weisend vor. Es ist in Form eines Mitnehmervorsprungs 168 ausgebildet, welcher wiederum korrespondierend zum Führungsdrahtmitnahmeglied 38 ausgebildet ist, nämlich zur Führungsdrahtausnehmung 48. In einer Mitnahmestellung stehen das Mitnehmerelement 166 und das Führungsdrahtmitnahmeglied 38 kraft- und/oder formschlüssig in Eingriff. Dies ist schematisch in den Figuren 2 und 3 dargestellt. In einer Einführstellung, die schematisch in Figur 11 dargestellt ist, stehen das Mitnehmerelement 166 und das Führungsdrahtmitnahmeglied 38 außer Eingriff.

Wie in Figur 3 gut zu erkennen, schließt sich der Längskanalabschnitt 164 proximalseitig an den Mitnehmervorsprung 168 an. Der Mitnehmervorsprung 168 ragt in der Mitnahmestellung teilweise in den Längskanal 24 hinein, sodass er in die Führungsdrahtausnehmung 48 eingreifen kann.

Das Mitnehmerelement 166 ist somit in radialer Richtung bezogen auf die Instrumentenlängsachse 18 bewegbar, nämlich verschiebbar, angeordnet beziehungsweise ausgebildet. Es ist zudem von der Mitnahmestellung entgegen der Wirkung einer vom Präparationsinstrument 10 umfassten Rückstelleinrichtung 170 in die Einführstellung bewegbar. Die Rückstelleinrichtung 170 umfasst ein Rückstellelement 172 in Form einer Feder 174. Die Feder 174 ist bei dem in den Figuren dargestellten Ausführungsbeispiel als Schraubenfeder ausgebildet. Alternativ kann sie auch in Form einer Blattfeder ausgebildet sein. Das Rückstellelement 172 stützt sich einerseits am Gehäuse 158 und andererseits am Mitnehmerglied 160 ab. Auf diese Weise kann die Rückstelleinrichtung 170 in radialer Richtung auf die Instrumentenlängsachse 18 hin wirken, mithin also das Mitnehmerglied 160 und damit das Mitnehmerelement 166 in Richtung auf die Instrumentenlängsachse 18 hin bewegen.

In einer Grundstellung nimmt die Mitnahmeeinrichtung 132 die beschriebene Mitnahmestellung ein. Das Rückstellelement 172 ist derart konfiguriert, dass das Mitnehmerelement 166 in der Mitnahmestellung unter Vorspannung gehalten ist.

Die beschriebene Mitnahmeeinrichtung 132 ist in Form einer Rast- beziehungsweise Schnappverbindungseinrichtung 176 ausgebildet. Zusammenwirkende Rast- beziehungsweise Schnappglieder der Rast- beziehungsweise Schnappverbindungseinrichtung 176 werden durch das Führungsdrahtmitnahmeglied 38 und das mit diesem zusammenwirkende Mitnehmerelement 166 gebildet.

Die Mitnahmeeinrichtung 132 umfasst ferner ein Betätigungsglied 178. Das Bestätigungsglied 178 ist in Form eines Vorsprungs ausgebildet, welcher in der Mitnahmestellung wie schematisch in Figur 3 dargestellt aus einer Gehäuseöffnung des Gehäuses 158 vorsteht. Das Betätigungsglied 178 weist eine von der Instrumentenlängsachse 18 weg weisende Betätigungsfläche 182 auf.

Wird auf die Betätigungsfläche 182 eine Betätigungskraft auf das Betätigungsglied 178 in Richtung auf die Instrumentenlängsachse 18 hin ausgeübt, wird das Mitnehmerglied 160 in Richtung der wirkenden Kraft bewegt und komprimiert die Feder 174. Durch die Bewegung des Mitnehmerglieds 160 in der beschriebenen Weise wird auch das Mitnehmerelement 166 in Richtung auf die Feder 174 hin bewegt, sodass der Mitnehmervorsprung 168 die Führungsdrahtausnehmung 48 und somit auch den Längskanal 24 freigibt. In dieser Einführstellung kann das Präparationsinstrument 10 relativ zum Führungsdraht 22 in axialer Richtung, also parallel zur Instrumentenlängsachse 18, verschoben werden, und zwar sowohl in distaler als auch in proximaler Richtung.

Das Betätigungsglied 178 und das Mitnehmerelement 166 sind beide am Mitnehmerglied 160 angeordnet beziehungsweise ausgebildet. Somit sind sie einstückig, nämlich monolithisch ausgebildet, denn das Mitnehmerglied 160 ist bei dem dargestellten Ausführungsbeispiel monolithisch ausgebildet.

Ferner sind der Führungsdraht 22 und die Mitnahmeeinrichtung 132 in der Mitnahmestellung bezogen auf die Instrumentenlängsachse 18 relativ zueinander verdrehbar. Dies wird dadurch erreicht, dass das Führungsdrahtmitnahmeglied 38 als Ringnut 52 ausgebildet ist. Der in die Führungsdrahtausnehmung 48 eingreifende Mitnehmervorsprung 168 verhindert jedoch eine axiale Relativbewegung zwischen der Mitnahmeeinrichtung 132 und damit dem proximalen Instrumententeil 30 einerseits und dem Führungsdraht 22 andererseits in der Mitnahmestellung.

Das Präparationsinstrument 10 ist wie bereits erläutert mit dem Führungsdraht 22 koppelbar ausgebildet. Insbesondere kann diese Kopplung genutzt werden zum Führen des Präparationsinstruments 10 beim Präparieren des Knochens 12.

Das Präparationsinstrument 10 umfasst ferner einen Führungsdrahtbewegungsmechanismus 184 zum Bewegen des Führungsdrahts 22 relativ zum distalen Ende 20 des Präparationsinstruments 10 in distaler und/oder proximaler Richtung.

Der Führungsdrahtbewegungsmechanismus 184 umfasst den distalen und den proximalen Instrumententeil 28, 30. Diese sind wie bereits erläutert relativ zueinander bewegbar, und zwar parallel zur Instrumentenlängsachse 18, ausgebildet. Der Führungsdrahtbewegungsmechanismus 184 umfasst die Bewegungsgewindeverbindung 32. Wird nämlich wie beschrieben der proximale Instrumententeil 30, der mit dem Führungsdraht 22 in der Mitnahmestellung gekoppelt ist, relativ zum distalen Instrumententeil 28 bewegt, so bewegt sich der Führungsdraht 22 mit dem proximalen Instrumententeil 30 mit. Somit ist der Führungsdrahtbewegungsmechanismus 184 in Form eines Vorschubmechanismus 186 zum Vorschieben des Führungsdrahts 22 relativ zum distalen Ende in distaler Richtung ausgebildet. Hierfür wird der proximale Instrumententeil 30 durch Hineinschrauben in den distalen Instrumententeil 28 in distaler Richtung bewegt, wobei der Führungsdraht 22 wie beschrieben dabei mitgenommen wird.

Bei dem in den Figuren dargestellten Ausführungsbeispiel des Präparationsinstruments 10 ist ein Innendurchmesser des Längskanals 24 im Bereich des proximalen Instrumententeils 30 einem Außendurchmesser des Führungsdrahts 22 entsprechend ausgebildet. Ein wenig Spiel ist vorgesehen, um eine Relativbewegung des proximalen Instrumententeils 30 und des Führungsdrahts 22 in der Einführstellung zu ermöglichen.

Ferner ist ein Innendurchmesser des Längskanals 24 im Bereich des Werkzeugkörpers 60 und somit im Bereich der Werkzeugelemente 62, 64 und 66, einem Außendurchmesser des Führungsdrahts 22 entsprechend ausgebildet. Auch hier ist ein wenig Spiel vorgesehen, um eine Bewegung des distalen Instrumententeils 28 relativ zum Führungsdraht 22 zu ermöglichen, und zwar sowohl in der Mitnahmestellung als auch in der Einführstellung. Der distale Instrumententeil 28 ist weder in der Mitnahmestellung noch in der Einführstellung mit dem Führungsdraht in axialer Richtung wirkend gekoppelt.

Die Ausgestaltung sowie Kopplung des distalen Instrumentengriffs 96, welcher ausgebildet ist zum Drehen des distalen Instrumententeils 28 um die Instrumentenlängsachse 18, wird nachfolgend näher erläutert.

Der distale Instrumentengriff 96 umfasst zwei Griffarme 188 und 190, welche in radialer Richtung bezogen auf die Instrumentenlängsachse 18 und diametral voneinander weg weisend angeordnet beziehungsweise ausgebildet sind. Der distale Instrumentengriff 96 ist mit dem distalen Instrumententeil 28 lösbar verbindbar ausgebildet. Er ist mit dem distalen Instrumententeil 28 in der bereits oben erläuterten Verbindungsstellung bezogen auf die Instrumentenlängsachse 18 zumindest in einer Drehrichtung drehfest gekoppelt.

Zum temporären Koppeln des distalen Instrumentengriffs 96 und des distalen Instrumententeils 28 dient die Verbindungseinrichtung 94 mit den zusammenwirkenden ersten und zweiten Verbindungselementen 90 und 92, die einerseits am distalen Instrumententeil 28 und andererseits am distalen Instrumentengriff 96 angeordnet beziehungsweise ausgebildet sind. Die ersten und zweiten Verbindungselemente 90, 92 stehen in der Verbindungstellung kraft- und/oder formschlüssig in Eingriff und in einer Reinigungsstellung außer Eingriff. In der Reinigungsstellung kann der distale Instrumentengriff 96 vollständig vom distalen Instrumententeil 28 abgenommen werden.

Der distale Instrumentengriff 96 ist in Form eines Ratschengriffs ausgebildet. Dies wird dadurch ermöglicht, dass die ersten und zweiten Verbindungselemente 90 und 92 in einer Drehstellung relativ zueinander um die Instrumentenlängsachse 18 in einer Drehrichtung verdrehbar sind. In einer entgegengesetzten Drehrichtung ist der distale Instrumentengriff 96 relativ zum distalen Instrumententeil 28 dagegen frei verdrehbar um die Instrumentenlängsachse 18. Diese Ratschenfunktion wird dadurch ermöglicht, dass der Verbindungsvorsprung 110 in der Drehstellung in radialer Richtung bezogen auf die Instrumentenlängsachse 18 auslenkbar angeordnet oder ausgebildet ist.

Die Verbindungseinrichtung 94 umfasst ferner einen Verbindungsring 192, welcher die in radialer Richtung weisende Verzahnung 106 umgibt. Der Verbindungsring 192 ist aufweitbar ausgebildet. Er umfasst einen sich in Umfangsrichtung bezogen auf die Instrumentenlängsachse erstreckenden Aufweitspalt 194. Der Verbindungsring 192 ist mit zwei freien, aufeinander zu weisenden Enden über zwei Griffarmstege 200 und 202 mit dem Griffarm 190 verbunden. Die beiden Griffarmstege 200 und 202 verlaufen parallel zueinander. Zwischen den beiden Griffarmstegen 200 und 202 erstreckt sich ein Federelement 204.

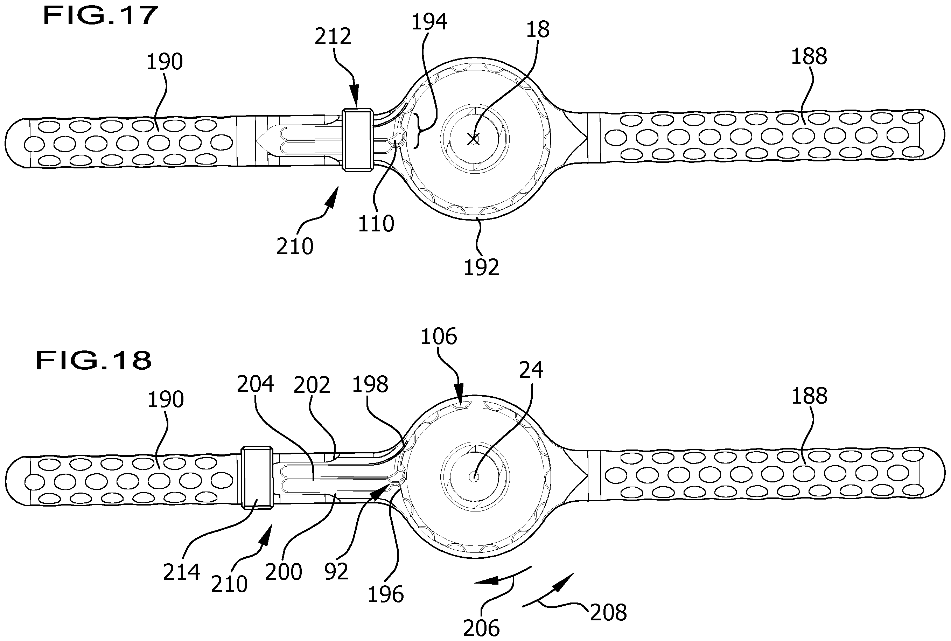

Der Verbindungsvorsprung 110 ist an einem freien Ende des Federelements 204 angeordnet. Das freie Ende des Federelements 204 weist in Richtung auf die Instrumentenlängsachse 18 hin. Die Länge des Federelements 204 ist so gewählt, dass der Verbindungsvorsprung 110 zwischen zwei Zähne 108 der Verzahnung 106 eingreifen kann. Der Verbindungsvorsprung 110 ist so ausgebildet, dass er am freien Ende 126, welches den Aufweitspalt 194 an einer Seite begrenzt, anliegt, wenn der distale Instrumentengriff 96 in Richtung des Pfeils 206, also in Uhrzeigerrichtung bei Draufsicht auf das distale Instrumententeil 28 wie in Figur 18 schematisch dargestellt verdreht wird. Bei dieser Drehrichtung nimmt der distale Instrumentengriff 96 den distalen Instrumententeil 28 mit. Bei einer Drehung des distalen Instrumentengriffs 96 im Gegenuhrzeigersinn, was durch den Pfeil 208 in Figur 18 symbolisiert wird, kann der Verbindungsvorsprung 110 jedoch aus einer Vertiefung zwischen zwei benachbarten Zähnen herausgleiten, was insbesondere durch das Federelement 204 ermöglicht wird. So lässt sich eine Orientierung der Griffarme 180 und 190 in gewünschter Weise einstellen, bevor in entgegengesetzter Drehrichtung wieder ein Drehmoment auf den distalen Instrumententeil 28 übertragen wird.

Der Griffarm 188 ist am Verbindungsring 192 dem Aufweitspalt 194 bezogen auf die Instrumentenlängsachse 18 diametral gegenüberliegend angeordnet.

Die Verbindungseinrichtung 94 umfasst ferner eine Sicherungseinrichtung 210 zum Sichern der ineinandergreifenden ersten und zweiten Verbindungselemente 90 und 92 in der Verbindungsstellung.

Die Sicherungseinrichtung 210 umfasst ein Sicherungselement 212. Dieses ist am distalen Instrumentengriff 96 von einer Entsicherungsstellung, in welcher der distale Instrumentengriff 96 und der distale Instrumententeil 28 in einer Einschraubdrehrichtung bezogen auf die Instrumentenlängsachse 18 verdrehbar sind, in eine Sicherungsstellung bewegbar, in welcher der distale Instrumentengriff 96 und der distale Instrumententeil 28 relativ zueinander in der Einschraubdrehrichtung bezogen auf die Instrumentenlängsachse unverdrehbar sind. Die Einschraubdrehrichtung wird hier definiert durch den Pfeil 206.

Die Entsicherungsstellung ist schematisch in Figur 18 dargestellt, die Sicherungsstellung in Figur 17. Das Sicherungselement 212 ist ausgebildet zum Verhindern eines Aufweitens des Verbindungsrings 192 in der Sicherungsstellung. Mithin wird also durch das Sicherungselement 212 in der Sicherungsstellung verhindert, dass sich der Aufweitspalt 194 vergrößert.

Das Sicherungselement 212 ist in Form einer Sicherungshülse 214 ausgebildet, welche auf dem Griffarm 190 in radialer Richtung bezogen auf die Instrumentenlängsachse 18, und zwar auf diese hin und von dieser weg, bewegbar ist, nämlich verschiebbar. Die Sicherungshülse 214 umschließt die Griffarmstege 200 und 202. In der Sicherungsstellung nimmt die Sicherungshülse 214 eine der Instrumentenlängsachse 18 maximal angenäherte Position ein wie schematisch in Figur 17 dargestellt und in der Entsicherungsstellung eine von der Instrumentenlängsachse 18 maximal entfernte Position wie schematisch in Figur 18 dargestellt.

Mit dem oben beschriebenen Ausführungsbeispiel des Präparationsinstruments 10 lässt sich ein Knochen 12 vor dem Verankern einer Knochenschraube 14 in einem Knochen oder einem Knochenmodel auf einfache Weise präparieren.

Hierfür wird das Präparationsinstrument 10 zunächst für seinen Einsatz vorbereitet. Der distale Instrumentengriff 96 wird mit dem distalen Instrumententeil 28 gekoppelt. Diesbezüglich sei verwiesen auf die Figuren 16 bis 18 samt zugehöriger obiger Beschreibung.

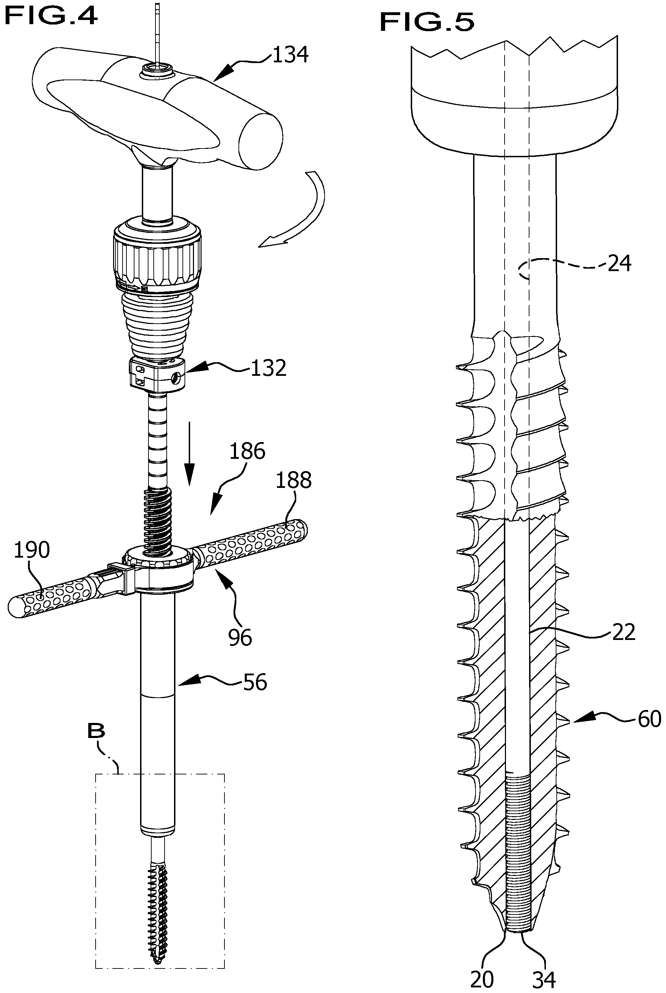

Der proximale Instrumentengriff 134 wird mit dem proximalen Instrumententeil 30 in der oben beschriebenen Weise gekoppelt. Der proximale Instrumententeil 30 kann nun mit dem Außengewindeabschnitt 118 in den Innengewindeabschnitt 116 des distalen Instrumententeils 28 eingeschraubt werden, wie dies schematisch in Figur 4 dargestellt ist.

Nun kann der Führungsdraht 22 entweder von distal oder von proximal herkommend in den Längskanal 24 eingeführt werden, bis er mit der Mitnahmeeinrichtung 132 gekoppelt ist. Wie beschrieben greift in der Mitnahmestellung das Mitnehmerelement 166 in die Führungsdrahtausnehmung 48 ein. Der proximale Instrumententeil 30 und der distale Instrumententeil 28 werden nun so relativ zueinander verschraubt, dass der in axialer Richtung relativ zum proximalen Instrumententeil 30 festgelegte Führungsdraht 22 mit seinem distalen Ende 34 bis an das distale Ende 20 heranreicht. Mithin steht also in dieser Ausgangsstellung der Führungsdraht 22 mit seinem distalen Ende 34 nicht über das distale Ende 20 des Präparationsinstruments 10 vor. Der Ausgewindeabschnitt 118 ragt in dieser Ausgangsstellung wie schematisch in den Figuren 4 und 5 dargestellt etwas in proximaler Richtung aus dem distalen Instrumententeil 28 vor.

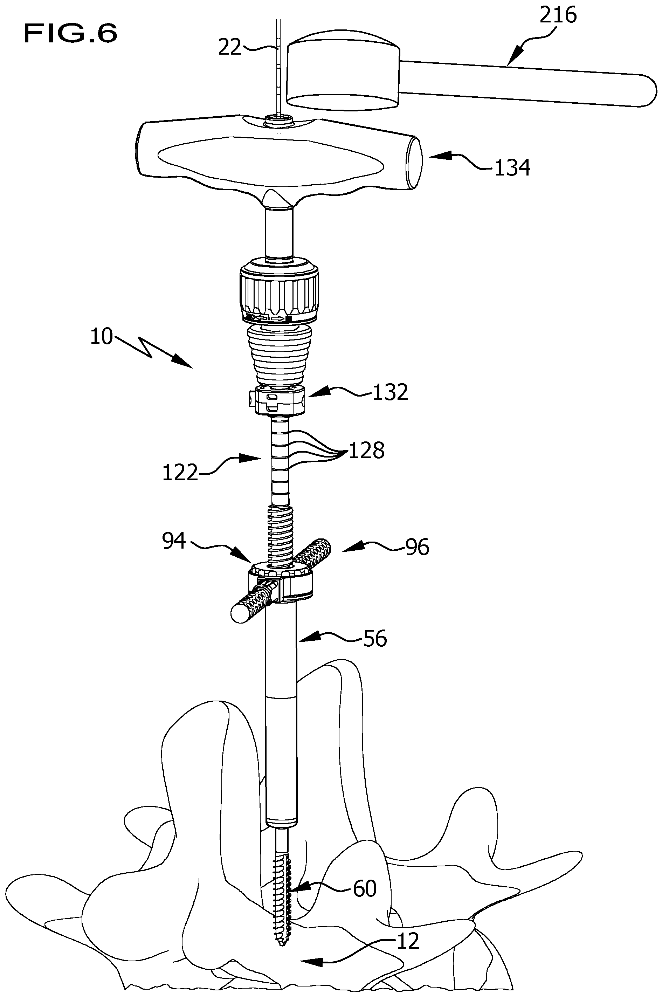

Nun kann mit dem Präparationsinstrument 10 der Knochen 12 präpariert werden. Hierzu wird zunächst das Perforationswerkzeug 22 eingesetzt, um die Kortikalis 74 zu perforieren. Hierzu kann insbesondere ein Schlagwerkzeug 216 eingesetzt werden, mit welchem auf das Präparationsinstrument 10 Schlagimpulse in distaler Richtung ausgeübt werden, und zwar indem mit dem Schlagwerkzeug, beispielsweise einem Schlitzhammer, auf den proximalen Instrumentengriff 134 eingeschlagen wird. Dies ist schematisch in Figur 6 dargestellt.

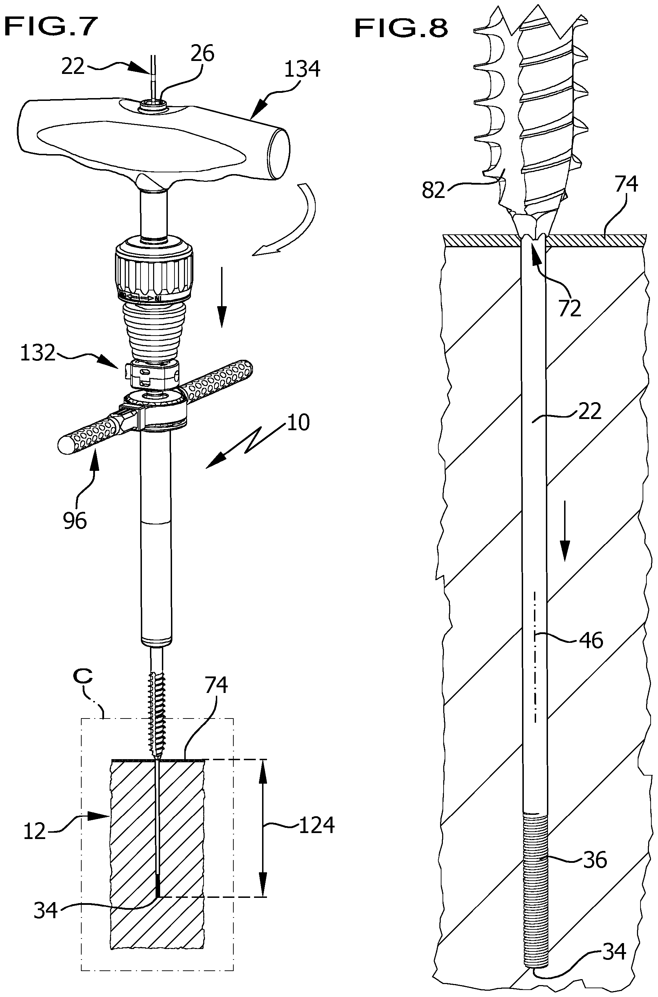

Ist die Kortikalis 74 wie beschrieben perforiert, kann nun der Führungsdraht 22 mit dem Präparationsinstrument 10 in den Knochen 12 eingetrieben werden. Hierfür wird der Führungsdrahtbewegungsmechanismus 184 genutzt. Das mit dem distalen Ende 20 am Knochen 12 anliegende Präparationsinstrument 10 wird vom Operateur dabei derart gehandhabt, dass er mit einer Hand den distalen Instrumententeil 28 festhält und mit seiner anderen Hand den proximalen Instrumentengriff 134 wie in Figur 7 dargestellt im Uhrzeigersinn verdreht. Dadurch wird der proximale Instrumententeil 30 in den distalen Instrumententeil 28 hineingeschraubt. Der mit dem proximalen Instrumententeil 30 gekoppelte Führungsdraht 22 wird dabei in den Knochen 12 hineinbewegt. Dies ist schematisch in den Figuren 7 und 8 dargestellt.

Das Einbringen des Führungsdrahts 22 erfolgt vorzugsweise unter Ultraschall- oder Röntgenkontrolle, um zu verhindern, dass Nerven oder Gefäße des Patienten verletzt werden.

Ist der Führungsdraht wie beschrieben kontrolliert im Knochen 12 platziert, kann an der Einführtiefenmesseinrichtung 122 abgelesen werden, wie weit das distale Ende 34 des Führungsdrahts 22 über das distale Ende 20 des distalen Instrumententeils 28 vorsteht. Mithin wird also der Abstand 124 bestimmt, und zwar durch Ablesen des Werts des gerade noch aus dem distalen Instrumententeils 28 herausstehenden und sichtbaren Rings 128 der Einführtiefenmesseinrichtung 122.

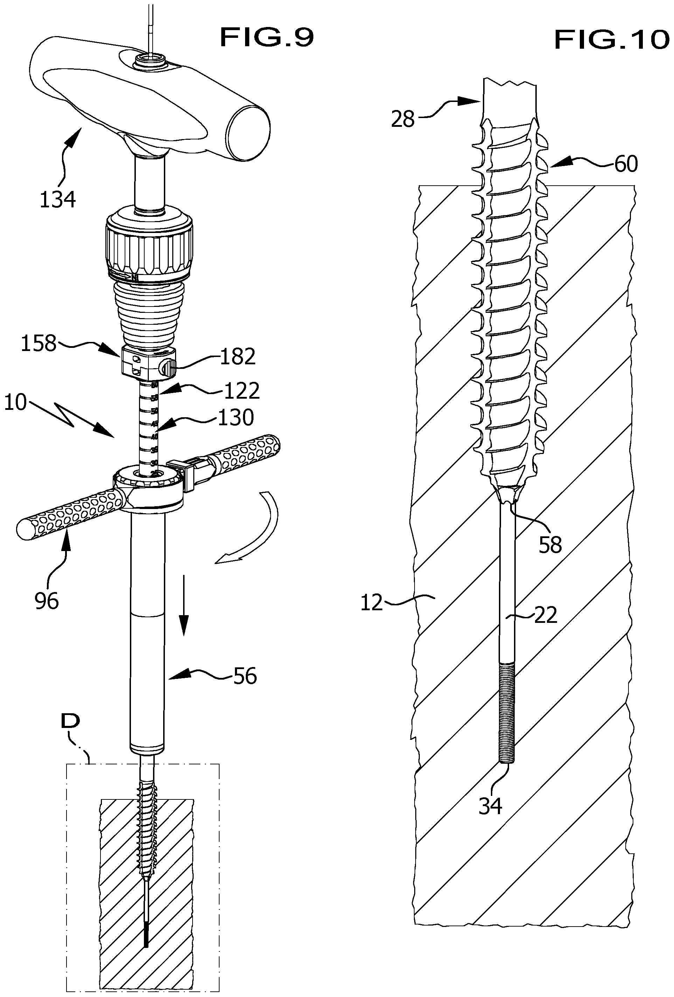

In einem nächsten Schritt kann das Schraubenloch 80 vorgebohrt werden. Hierfür dient das Bohrwerkzeug 78. Der Operateur hält nun den proximalen Instrumententeil 30 am proximalen Instrumentengriff 134 fest und dreht den distalen Instrumententeil 28 durch Verdrehen des distalen Instrumentengriffs 96 im Uhrzeigersinn relativ zum proximalen Instrumententeil 30. Durch diese schematisch in Figur 9 dargestellte Drehbewegung des distalen Instrumententeils 28 wird der Abstand 124 zwischen dem distalen Ende 34 des Führungsdrahts 22 und dem distalen Ende 20 des Perforationsinstruments 10 wieder verringert. Der Führungsdraht 20 führt dabei den Werkzeugkörper 60. Die Bohrschneiden 82 schneiden das Schraubenloch 80 bei dieser Schraubbewegung des distalen Instrumententeils 28 relativ zum proximalen Instrumententeil 30 in den Knochen 12 hinein. Bei diesem Einschrauben wird gleichzeitig auch das Gewinde 86 in das Schraubenloch 80 eingebracht, und zwar mit dem Gewindeschneidwerkzeug 84, welches ebenfalls am Werkzeugkörper 60 ausgebildet ist. Die Figuren 9 und 10 zeigen schematisch das Formen des Schraubenlochs sowie das Einbringen des Gewindes 86 in den Knochen 12.

Der Knochen 12 ist nun mit dem Präparationsinstrument 10 so weit präpariert, dass die Knochenschraube 14 platziert werden kann. Um die Knochenschraube 14 zuverlässig an die gewünschte Position im Knochen heranzuführen, wird das Präparationsinstrument 10 aus dem Knochen 12 entfernt. Hierzu wird der distale Instrumententeil 28 in umgekehrter Richtung aus dem Knochen 12 herausgedreht. Sobald der Werkzeugkörper 60 vollständig aus dem Knochen 12 herausbewegt ist, wird das Präparationsinstrument 10 vom Führungsdraht 22 abgekoppelt, indem eine Betätigungskraft auf die Betätigungsfläche 182 des Betätigungsglieds 178 der Mitnahmeeinrichtung 132 ausgeübt wird. Das Mitnehmerelement 166 gibt dadurch die Führungsdrahtausnehmung 48 frei. Das Präparationsinstrument 10 kann in dieser Einführstellung in proximaler Richtung vom Führungsdraht 22 abgezogen werden.

Der Führungsdraht 22 verbleibt in der in Figur 11 dargestellten Position im Knochen 12. Er dient der Führung der Knochenschraube 14, die durchgehend kanüliert ist und von proximal her kommend über das proximale Ende 42 des Führungsdrahts 22 geschoben wird. Die Knochenschraube 14 kann bis an das Schraubenloch 80 herangeführt werden. Sie wird dann mit einem Einschraubwerkzeug 218, welches ebenfalls über den Führungsdraht 22 von proximal her kommend geschoben wird, in den Knochen 12, nämlich in das vorbereitete Schraubenloch 80, eingeschraubt werden.

Ist die Knochenschraube 14 in gewünschter Weise im Knochen 12 platziert, werden sowohl das Einschraubwerkzeug 218 als auch der Führungsdraht 22 in proximaler Richtung von der Knochenschraube 14 entfernt.

Wie beschrieben ist es mit dem Präparationsinstrument 10, welches als sogenanntes "All-in-one"-Instrument ausgebildet ist, ein Knochen 12 vor dem Verankern einer Knochenschraube 14 präpariert werden, und zwar durch Platzieren des Führungsdrahts 22 im Knochen, Ausbilden eines Schraubenlochs 18, optional Versehen desselben mit einem Gewinde 86, Messen der erforderlichen Schraubenlänge der Knochenschraube 14 und Entfernen des Präparationsinstruments 10 mit im Knochen 12 verbleibendem Führungsdraht 22. Das Präparationsinstrument 10 ermöglicht die Durchführung aller beschriebenen Schritte ohne Einsatz weiterer Instrumente. So kann die Präparation des Knochens 12 für einen Operateur vereinfacht werden. Eine Operationszeit lässt sich dadurch verkürzen, was einen Eingriff für einen Patienten insgesamt schonender macht.

### Bezugszeichenliste

- 10: Präparationsinstrument
- 12: Knochen
- 14: Knochenschraube
- 16: Instrumentenschaft
- 18: Instrumentenlängsachse
- 20: distales Ende
- 22: Führungsdraht
- 24: Längskanal
- 26: proximales Ende
- 28: distaler Instrumententeil
- 30: proximaler Instrumententeil
- 32: Bewegungsgewindeverbindung
- 34: distales Ende
- 36: Außengewindeabschnitt
- 38: Führungsdrahtmitnahmeglied
- 40: Abstand
- 42: proximales Ende
- 44: Abstand
- 46: Führungsdrahtlängsachse
- 48: Führungsdrahtausnehmung
- 50: Nut
- 52: Ringnut
- 54: Markierung
- 56: distaler Schaft
- 58: distales Ende
- 60: Werkzeugkörper
- 62: Werkzeugelement
- 64: Werkzeugelement
- 66: Werkzeugelement
- 68: Außendurchmesser
- 70: Werkzeugaußendurchmesser
- 72: Perforationswerkzeug
- 74: Kortikalis
- 76: Spitze
- 78: Bohrwerkzeug
- 80: Schraubenloch
- 82: Bohrschneide
- 84: Gewindeschneidwerkzeug
- 86: Gewinde
- 88: Schneidgewinde
- 90: erstes Verbindungselement
- 92: zweites Verbindungselement
- 94: Verbindungseinrichtung
- 96: distaler Instrumentengriff
- 98: Körper
- 100: Ringflansch
- 102: distaler Anschlag
- 104: distale Anschlagfläche
- 106: Verzahnung
- 108: Zahn
- 110: Verbindungsvorsprung
- 112: Gewindeabschnitt
- 114: Gewindeabschnitt
- 116: Innengewindeabschnitt
- 118: Außengewindeabschnitt
- 120: distales Ende
- 122: Einführtiefenmesseinrichtung
- 124: Abstand
- 126: Markierungselement
- 128: Ring
- 130: Beschriftung
- 132: Mitnahmeeinrichtung
- 134: proximaler Instrumentengriff
- 136: Kopplungseinrichtung
- 138: erstes Kopplungselement
- 140: zweites Kopplungselement
- 142: Kopplungsvorsprung
- 144: Kopplungsausnehmung
- 146: Außenmehrkant
- 148: proximaler Anschlag
- 150: proximale Anschlagfläche
- 152: Hülse
- 154: proximales Ende
- 156: Griffelement
- 158: Gehäuse
- 160: Mitnehmerglied
- 162: Durchgangsöffnung
- 164: Längskanalabschnitt
- 166: Mitnehmerelement
- 168: Mitnehmervorsprung
- 170: Rückstelleinrichtung
- 172: Rückstellelement
- 174: Feder
- 176: Rast- oder Schnappverbindungseinrichtung
- 178: Betätigungsglied
- 180: Gehäuseöffnung
- 182: Betätigungsfläche
- 184: Führungsdrahtbewegungsmechanismus
- 186: Vorschubmechanismus
- 188: Griffarm
- 190: Griffarm
- 192: Verbindungsring
- 194: Aufweitspalt
- 196: freies Ende
- 198: freies Ende
- 200: Griffarmsteg
- 202: Griffarmsteg
- 204: Federelement
- 206: Pfeil
- 208: Pfeil
- 210: Sicherungseinrichtung
- 212: Sicherungselement
- 214: Sicherungshülse
- 216: Schlagwerkzeug
- 218: Einschraubwerkzeug

## Patentansprüche

1. Präparationsinstrument (10) zum Präparieren eines Knochens (12) vor der Verankerung einer Knochenschraube (14), insbesondere einer Pedikelschraube, im Knochen (12), wobei das Präparationsinstrument (10) einen eine Instrumentenlängsachse (18) definierenden Instrumentenschaft (16), ein distales Ende (20) und einen vom Instrumentenschaft (16) separaten und vollständig trennbaren Führungsdraht (22), insbesondere in Form eines K-Drahts, umfasst und wobei das Präparationsinstrument (10) ausgebildet ist zum Verankern des Führungsdrahts (22) im Knochen (12), **dadurch gekennzeichnet, dass** der Führungsdraht (22) mit dem Präparationsinstrument (10) koppelbar ausgebildet ist, insbesondere zum Führen des Präparationsinstruments (10) beim Präparieren des Knochens (12), und dass das Präparationsinstrument (10) einen Führungsdrahtbewegungsmechanismus (184) umfasst zum Bewegen des Führungsdrahts (22) relativ zum distalen Ende (20) in distaler und/oder proximaler Richtung.

2. Präparationsinstrument nach Anspruch 1, **dadurch gekennzeichnet, dass** das Präparationsinstrument (10) einen sich parallel zur Instrumentenlängsachse (18) erstreckenden, das Präparationsinstrument (10) durchsetzenden Längskanal (24) umfasst.

3. Präparationsinstrument nach einem der voranstehenden Ansprüche, **dadurch gekennzeichnet, dass** das Präparationsinstrument (10) einen distalen und einen proximalen Instrumententeil (28, 30) umfasst, dass der Führungsdrahtbewegungsmechanismus (184) den distalen und den proximalen Instrumententeil (28, 30) umfasst und dass der distale und der proximale Instrumententeil (28, 30) relativ zueinander bewegbar, insbesondere parallel zur Instrumentenlängsachse (18), ausgebildet sind, wobei insbesondere der Führungsdrahtbewegungsmechanismus (184) eine Bewegungsgewindeverbindung (32) umfasst und dass der distale und der proximale Instrumententeil (28, 30) über die Bewegungsgewindeverbindung (32) miteinander verschraubbar koppelbar sind,
wobei weiter insbesondere die Bewegungsgewindeverbindung (32) zwei zusammenwirkende Gewindeabschnitte (112, 114) umfasst, welche in Form eines Innengewindeabschnitts (116) und eines zu diesem korrespondierenden Außengewindeabschnitts (118) ausgebildet sind, und dass der eine der zwei Gewindeabschnitte (112, 114) am distalen Instrumententeil (28) angeordnet oder ausgebildet ist und dass der andere der zwei Gewindeabschnitte (112, 114) am proximalen Instrumententeil (30) angeordnet oder ausgebildet ist.

4. Präparationsinstrument nach einem der voranstehenden Ansprüche, **dadurch gekennzeichnet, dass** der Führungsdrahtbewegungsmechanismus (184) eine Mitnahmeeinrichtung (132) umfasst, dass die Mitnahmeeinrichtung (132) und der Führungsdraht (22) in einer Mitnahmestellung kraft- und/oder formschlüssig bezogen auf die Instrumentenlängsachse (18) in axialer Richtung gekoppelt in Eingriff und in einer Einführstellung außer Eingriff stehen.

5. Präparationsinstrument nach Anspruch 4, **dadurch gekennzeichnet, dass** die Mitnahmeeinrichtung (132) ein Mitnehmerelement (166) umfasst, welches in der Mitnahmestellung mit einem Führungsdrahtmitnahmeglied (38) des Führungsdrahts kraft- und/oder formschlüssig in Eingriff steht und in einer Einführstellung außer Eingriff steht.

6. Präparationsinstrument nach Anspruch 5, **dadurch gekennzeichnet, dass** das Mitnehmerelement (166) von der Mitnahmestellung entgegen der Wirkung einer vom Präparationsinstrument (10) umfassten Rückstelleinrichtung (170) in die Einführstellung bewegbar ist,
wobei insbesondere die Rückstelleinrichtung (170) mindestens ein Rückstellelement (172) umfasst.

7. Präparationsinstrument nach Anspruch 5 oder 6, **dadurch gekennzeichnet, dass** die Mitnahmeeinrichtung (132) am proximalen Instrumententeil (30) angeordnet oder ausgebildet ist.

8. Präparationsinstrument nach einem der voranstehenden Ansprüche,
**dadurch gekennzeichnet, dass** das Präparationsinstrument (10) mindestens ein Werkzeugelement (62, 64, 66) zum Präparieren des Knochens (12) umfasst,
wobei insbesondere das mindestens eine Werkzeugelement (62, 64, 66)
a) am distalen Instrumententeil (28) in distaler Richtung weisend angeordnet oder ausgebildet ist
und/oder
b) in Form eines Perforationswerkzeugs (72) zum Perforieren der Kortikalis (74) des Knochens (12) ausgebildet ist
und/oder
c) in Form eines Bohrwerkzeugs (78) zum Bohren eines Schraubenlochs (80) im Knochen (12) ausgebildet ist
und/oder
d) in Form eines Gewindeschneidwerkzeugs (84) zum Schneiden eines Gewindes (86) in das Schraubenloch (80) im Knochen (12) ausgebildet ist.

9. Präparationsinstrument nach einem der voranstehenden Ansprüche,
**dadurch gekennzeichnet, dass** das Präparationsinstrument (10) eine Einführtiefenmesseinrichtung (122) umfasst zum Bestimmen eines Abstands (124) des distalen Endes (24) des aus dem distalen Ende (20) des Präparationsinstruments (10) vorstehenden Führungsdrahts (22) vom distalen Ende (20) des Präparationsinstruments (10),
wobei insbesondere die Einführtiefenmesseinrichtung (122) eine Mehrzahl von Markierungselementen (126) umfasst und dass die Markierungselemente (126) proximalseitig des vom proximalen Instrumententeil (30) umfassten Gewindeabschnitts (114) am proximalen Instrumententeil (30) angeordnet oder ausgebildet sind.

10. Präparationsinstrument nach einem der Ansprüche 3 bis 9, **dadurch gekennzeichnet, dass** das Präparationsinstrument (10) einen distalen Instrumentengriff (96) umfasst zum Drehen des distalen Instrumententeils (28) um die Instrumentenlängsachse (18).

11. Präparationsinstrument nach einem der Ansprüche 3 bis 10, **dadurch gekennzeichnet, dass** das Präparationsinstrument (10) einen proximalen Instrumentengriff (134) umfasst zum Drehen des proximalen Instrumententeils (30) um die Instrumentenlängsachse (18),
wobei insbesondere der proximale Instrumentengriff (134) mit dem proximalen Instrumententeil (30) in einer Kopplungsstellung bezogen auf die Instrumentenlängsachse (18) drehfest gekoppelt ist.

12. Präparationsinstrument nach Anspruch 11, **dadurch gekennzeichnet, dass** das Präparationsinstrument (10) eine Kopplungseinrichtung (136) umfasst zum kraft- und/oder formschlüssigen Koppeln des proximalen Instrumentengriffs (134) und des proximalen Instrumententeils (30) in der Kopplungsstellung.

13. Präparationsinstrument nach einem der Ansprüche 3 bis 12, **dadurch gekennzeichnet, dass** ein Innendurchmesser des Längskanals (24) im Bereich des proximalen Instrumententeils (30) einem Außendurchmesser des Führungsdrahts (22) entspricht.

14. Präparationsinstrument nach einem der Ansprüche 8 bis 13, **dadurch gekennzeichnet, dass** ein Innendurchmesser des Längskanals (24) im Bereich des mindestens einen Werkzeugelements (62, 64, 66) einem Außendurchmesser des Führungsdrahts (22) entspricht.

15. Verfahren zum Präparieren eines künstlichen Knochenmodells vor der Verankerung einer Knochenschraube (14), insbesondere einer Pedikelschraube, bei welchem Verfahren ein Schraubenloch (80) im Knochenmodell präpariert wird, **dadurch gekennzeichnet, dass** zum Präparieren ein Präparationsinstrument (10) nach einem der voranstehenden Ansprüche verwendet wird.
